(19) Europäisches Patentamt / European Patent Office / Office européen des brevets

(11) **EP 3 887 800 B1**

(12) **EUROPEAN PATENT SPECIFICATION**

(45) Date of publication and mention
of the grant of the patent:
**17.04.2024 Bulletin 2024/16**

(21) Application number: **19809491.4**

(22) Date of filing: **29.11.2019**

(51) International Patent Classification (IPC):
***G01N 21/31*** *(2006.01)*

(52) Cooperative Patent Classification (CPC):
**G01N 21/31**

(86) International application number:
**PCT/EP2019/083152**

(87) International publication number:
**WO 2020/109589 (04.06.2020 Gazette 2020/23)**

(54) **METHOD FOR DETERMINING ONE CONTENT IN PROTEIN AND ASSOCIATED DEVICES AND METHODS**

VERFAHREN ZUR BESTIMMUNG EINES PROTEINGEHALTS UND ZUGEHÖRIGE VORRICHTUNGEN UND VERFAHREN

PROCÉDÉ DE DÉTERMINATION D'UN CONTENU DANS UNE PROTÉINE ET DISPOSITIFS ET PROCÉDÉS ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2018 EP 18306588**

(43) Date of publication of application:
**06.10.2021 Bulletin 2021/40**

(73) Proprietors:
• **INSTITUT NATIONAL DE LA SANTE ET DE LA RECHERCHE MEDICALE - INSERM**
**75013 Paris (FR)**
• **Université Paris-Est Créteil Val de Marne**
**94010 Creteil Cedex (FR)**
• **ASSISTANCE PUBLIQUE, HOPITAUX DE PARIS**
**75004 Paris (FR)**

(72) Inventors:
• **KIGER, Laurent**
**94017 Creteil Cedex (FR)**
• **BARTOLUCCI, Pablo**
**94017 Creteil Cedex (FR)**
• **MARDEN, Michael**
**94017 Creteil Cedex (FR)**

(74) Representative: **Icosa**
**83 avenue Denfert-Rochereau**
**75014 Paris (FR)**

(56) References cited:
**US-A1- 2006 034 730     US-A1- 2017 045 441**
**US-B1- 6 911 427**

**Description**

TECHNICAL FIELD OF THE INVENTION

The present invention concerns a method for determining at least one content in protein. The invention also relates to a method for diagnosing a heme-related or hemoprotein-related disorder. The invention also concerns a method for identifying a therapeutic target for preventing and/or treating a heme-related or hemoprotein-related disorder. The invention also relates to a method for identifying a biomarker, the biomarker being a diagnostic biomarker of a heme-related or hemoprotein-related disorder, a susceptibility biomarker of a liver disease, a prognostic biomarker of a heme-related or hemoprotein-related disorder or a predictive biomarker in response to the treatment of a heme-related or hemoprotein-related disorder. The invention also concerns a method for screening a compound useful as a medicine, the compound having an effect on a known therapeutical target, for preventing and/or treating a heme-related or hemoprotein-related disorder. The method also concerns a method for qualifying or disqualifying medical bags and a method for monitoring a treatment against a heme-related or hemoprotein-related disorder. The invention also relates to the associated computer program products and a computer readable medium.

BACKGROUND OF THE INVENTION

Many illnesses exhibit symptoms that affect the blood.

It is thus desirable that the blood be analyzed chemically.

It is known from document US 9 638 686 A1 a method of measuring whole-blood hemoglobin parameters includes providing a light source, guiding light having the spectral range from the light source along an optical path, providing a cuvette module with a sample receiving chamber, providing a pair of first and second optical diffusers disposed in the optical path where the cuvette module is disposed between the pair of first and second optical diffusers, guiding light from the cuvette module into an optical spectrometer, and processing an electrical signal from the spectrometer into an output signal useable for displaying and reporting hemoglobin parameter values and/or total bilirubin parameter values of the sample of whole blood.

It is known from document US 9 638 686 A1 a method of measuring whole-blood hemoglobin parameters includes providing a light source, guiding light having the spectral range from the light source along an optical path, providing a cuvette module with a sample receiving chamber, providing a pair of first and second optical diffusers disposed in the optical path where the cuvette module is disposed between the pair of first and second optical diffusers, guiding light from the cuvette module into an optical spectrometer, and processing an electrical signal from the spectrometer into an output signal useable for displaying and reporting hemoglobin parameter values and/or total bilirubin parameter values of the sample of whole blood.

Document US 2007/292963 A1 also suggests a method for determining the presence and concentration of components in serum includes the steps of providing a serum sample, performing optical analysis of the serum sample using a spectrometer to provide spectral data based on optical properties of the serum sample, determining the concentrations of albumin, globulins and hemoglobin in the serum sample based on comparisons of the spectral data with reference spectra and outputting the determined concentrations of the albumin, globulins and hemoglobin. The invention can be used to accurately determine the concentration of albumin, globulins and hemoglobin in blood serum and can form the basis of initial cancer screening and to gauge a patient's response to treatment.

It is also known from document EP 1 211 505 A1 a method for measuring substances which carries out both sample preparation and detection of substances in a sample, in accordance with the photothermal conversion detection method, in a capillary of a microchip, whereby the quantity of substances, such as hemoglobin and Aluminium phosphide (ALP), can be measured from a very small amount of sample obtained from the constituents of living organism simply and easily and for a very short period time. In addition, the method allows the wastes caused by the measurements to be small. Further, the method of this invention employs laser light having a long wave length as excitation light, whereby the photothermal conversion detection device can be manufactured and the measurements can be carried out at low costs. Thus, the method for measuring substances of document EP 1 211 505 A1 can be suitably applied to the POC analyses and the like. Further, the method for measuring substances of this invention allows even a blood sample having chyle therein to be measured simply and easily in accordance with the photothermal detection method. Still further, the use of the measuring reagent of this invention allows the quantity of substances, such as hemoglobin and ALP, in a very small amount of sample obtained from the constituent of living organism to be measured stably in accordance with the photothermal converting detection method.

Document US 2006/034730 A1 concerns an iterative method for determining at least one content in protein in a biological sample, several proteins among which oxyhemoglobin, methemoglobin, and bilirubin. In particular, US 2006/034730 A1 discloses a method exploiting spectroscopy and dosing techniques to determine accurately the content in proteins of the biological sample, notably for oxyhemoglobin, methemoglobin, heme bound to serum albumin and hemopexin and bilirubin. Such method can be used in various applications concerning heme-related or hemoprotein-related disorders, notably method for diagnosing, for following a treatment, for determining biomarkers or for screening.

However, the method and apparatus of these documents are not easy to implement and provide with measurements that are not as accurate as may be desired.

SUMMARY OF THE INVENTION

The invention aims at providing a method for determining at least one protein in the blood which is more precise while still being simple to implement.

To this end, the specification describes a method for determining at least one content in protein in a biological sample, several proteins among which oxyhemoglobin, methemoglobin, heme bound to serum albumin, heme bound to hemopexin (total hemopexin) and bilirubin as defined in appended claim 1.

According to further aspects of the invention which are advantageous but not compulsory, the determining method might incorporate one or several of the following features, taken in any technically admissible combination:

- step c) is iterated by decreasing values of second derivatives of the proteins.

A simulation over a wavelength range of the second derivative is used in particular when two species contribute to the signal.

At the least one of the following properties is fulfilled :

- a spectral analysis is carried out for the oxyhemoglobin and the normalization coefficient is calculated for at least one wavelength chosen in three intervals, the first interval encompassing wavelengths comprised between 415 nanometers and 417 nanometers, the second interval encompassing wavelengths comprised between 542 nanometers and 544 nanometers and a third interval encompassing wavelengths comprised between 576 nanometers and 578 nanometers, the interval being preferably the third interval;
- a spectral analysis is carried out for the methemoglobin and the normalization coefficient is calculated for at least one wavelength chosen in the range of 350 nanometers to 750 nanometers, and
- a spectral analysis is carried out for the bilirubin and the normalization coefficient is calculated for at least one wavelength chosen in the range of 350 nanometers to 750 nanometers.

- a spectral analysis is carried out for the plasma heme and the normalization coefficient is calculated for at least one wavelength chosen in the range of 300 nanometers to 750 nanometers using heme bound to serum albumin and heme bound to hemopexin reference spectra.

At the step for outputting, the determined contents comprise additional contents in protein which are different from the content in oxyhemoglobin, from the content in methemoglobin, from the content in heme bound to serum albumin and hemopexin from the content in bilirubin, the additional contents being the contents of proteins chosen in the group consisting of:

- carboxylated hemoglobin,
- ferryl hemoglobin,
- other heme target molecules
- myoglobin,
- porphyrins,
- products of catabolism of heme by heme oxygenase,
- products of bilirubin degradation and oxidation, such as biliverdin or heme boxes, stercobilin, urobillin,
- products of organ dysfunction and cytosis,
- metabolic substrates,
- cofactors, and
- metabolic degradation products.

- when the content in oxyhemoglobin, methemoglobin, heme bound to serum albumin and bilirubin are successively determined, at least one of oxyhemoglobin, methemoglobin, heme bound to serum albumin and bilirubin are determined by a chemical dosing.

The specification describes a method for predicting that a subject is at risk of suffering from a heme-related or hemoprotein-related disorder, the method for predicting at least comprising the step of:

- carrying out the steps of a determining method at least one content in protein in a biological sample of the subject, to obtain determined parameters, the determining method being as previously described, and
- predicting that the subject is at risk of suffering from the heme-related or hemoprotein-related disorder on the determined parameters.

The specification also relates to a method for diagnosing a heme-related or hemoprotein-related disorder, the method for diagnosing at least comprising the step of:

- carrying out the steps of a determining method at least one content in protein in a biological sample of the subject, to obtain determined contents in protein, the determining method being as previously described, and
- diagnosing the heme-related or hemoprotein-related disorder based on the determined contents in protein.

The specification describes a method for identifying a therapeutic target for preventing and/or treating a heme-related or hemoprotein-related disorder, the method comprising the steps of:

- carrying out the steps of a method for determining at least one content in protein in a biological sample of images of a first subject, to obtain first determined contents in protein, the determining method being as previously described and the first subject being a subject suffering from the heme-related or hemoprotein-related disorder,
- carrying out the steps of the method for determining at least one content in protein in a biological sample of a second subject, to obtain second determined contents in protein, the determining method being as previously described and the second subject being a subject not suffering from the heme-related or hemoprotein-related disorder, and
- selecting a therapeutic target based on the comparison of the first and second determined contents in protein.

The specification also relates to a method for defining stages of a heme-related or hemoprotein-related disorder, the method for defining at least comprising the step of:

- carrying out the steps of a method for determining at least one content in protein in a biological sample of the subject, to obtain determined contents in protein, the determining method being as previously described, and
- defining the stages of the heme-related or hemoprotein-related disorder based on the determined contents in protein as well as in association with other biological parameters (predictive algorithm for a diagnosis).

The specification also concerns a method for screening a compound useful as a medicine, the compound having an effect on a known therapeutical target, for preventing and/or treating a heme-related or hemoprotein-related disorder, the method comprising the steps of:

- carrying out the steps of a method for determining at least one content in protein in a biological sample of a first subject, to obtain first determined contents in protein, the determining method being as previously described and the first subject being a subject suffering from the heme-related or hemoprotein-related disorder and having received the compound,
- carrying out the steps of the method for determining at least one content in protein in a biological sample of the second subject, to obtain second determined contents in protein, the determining method being as previously described and the second subject being a subject suffering from the heme-related or hemoprotein-related disorder and not having received the compound, and
- selecting a compound based on the comparison of the first and second determined contents in protein.

The specification describes a method for qualifying or disqualifying medical bags containing a biological sample of subject, the method comprising:

- carrying out the steps of a method for determining at least one content in protein in the medical bag, to obtain determined contents in protein, the determining method being as previously described, and
- qualifying or disqualifying medical bags based on the determined contents in protein.

The specification also concerns a method for identifying a biomarker, the biomarker being a diagnostic biomarker of a heme-related or hemoprotein-related disorder, a susceptibility biomarker of a heme-related or hemoprotein-related disorder, a prognostic biomarker of a heme-related or hemoprotein-related disorder or a predictive biomarker in response to the treatment of a heme-related or hemoprotein-related disorder, the method comprising the steps of:

- carrying out the steps of a method for determining at least one content in protein in a biological sample of a first subject, to obtain first determined contents, the determining method being as previously described and the first subject being a subject suffering from the heme-related or hemoprotein-related disorder,

- carrying out the steps of the method for determining at least one content in protein in a biological sample of images of a second subject, to obtain second determined contents in protein, the determining method being as previously described and the second subject being a subject not suffering from the heme-related or hemoprotein-related disorder, and
- selecting a biomarker based on the comparison of the first and second determined contents in protein.

The specification also concerns a method for monitoring a treatment against heme-related or hemoprotein-related disorder in a subject suffering from the heme-related or hemoprotein-related disorder and having received the treatment, the method comprising:

- carrying out the steps of a method for determining at least one content in protein in a biological sample of the subject, to obtain determined contents in protein, the determining method being as previously described, and
- monitoring the determined contents in protein with monitoring a treatment against hemoglobin related disease in a subject suffering from the heme-related or hemoprotein-related disorder and having received the treatment.

The specification also describes a computer program product comprising a computer readable medium, having thereon a computer program comprising program instructions, the computer program being loadable into a data-processing unit and adapted to cause execution of steps of a method as previously described when the computer program is run by the data processing unit.

The specification also relates to a computer readable medium having encoded thereon a computer program as previously described.

The specification also concerns an apparatus for determining at least one content in protein in a biological sample, several proteins among which oxyhemoglobin, methemoglobin, heme bound to serum albumin, heme bound or free hemopexin (total concentration) and bilirubin, the apparatus for determining at least comprising a spectrophotometer, dosing materials and an analysis system, the apparatus being adapted to carry out one method as previously described.

BRIEF DESCRIPTION OF THE DRAWINGS

The invention will be better understood on the basis of the following description which is given in correspondence with the annexed figures and as an illustrative example, without restricting the object of the invention. In the annexed figures:

- figure 1 shows schematically an example of apparatus adapted to determine at least one content in protein in a biological sample;
- figure 2 shows an example of system and computer program product which are part of the apparatus and whose interaction enables to carry out calculations used by the apparatus in order to determine the content(s) in protein of the sample,
- figure 3 is a flowchart of an example of carry out an example of method for determining at least one content in protein in the biological sample,
- figure 4 is a graph showing various absorption spectra for different heme complexes,
- figure 5 is a graph showing different second derivatives for different heme complexes, and
- figure 6 is a graph showing a calibrating measurement based on hemopexin measurements.

DETAILED DESCRIPTION OF SOME EMBODIMENTS

DESCRIPTION OF THE APPARATUS

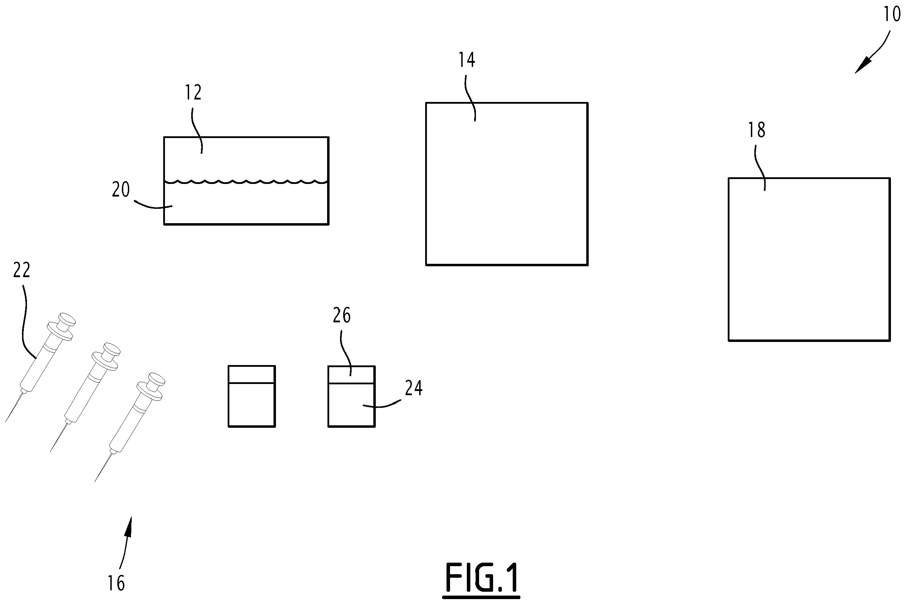

An apparatus 10 is represented on figure 1.

The apparatus 10 is an apparatus adapted for determining at least one content protein in a biological sample.

The apparatus 10 comprises at least one container 12, a spectrophotometer 14, dosing materials 16 and an analysis system 18.

The at least one container 12 comprises a biological sample 20 to analyze.

The biological sample 20 is a sample from a subject

The subject is an animal such as a mammal, like a mouse, a monkey or a human being.

As used herein and according to all aspects of the invention, the term "sample" denotes, blood, fresh whole blood, peripheral-blood, peripheral blood mononuclear cell (PBMC), serum, plasma or urine.

The sample 20 comprises several proteins among which oxyhemoglobin, methemoglobin, heme bound to serum albumin, heme bound to or free hemopexin and bilirubin.

Oxyhemoglobin designates a reduced (Fe2+) form of hemoglobin. In this reduced form, the hemoglobin is a hemoglobin whose reduced heme ($Fe^{2+}$) is bound to oxygen under air (unbound deoxygenated under low $O_2$ pressure).

The oxyhemoglobin encompasses oxyhemoglobin A (formula $HbAO_2$), oxyhemoglobin F (formula $HbFO_2$), oxyhemoglobin A2, oxy glycated hemoglobin as well as other widespread hemoglobin variants (S, E, C).

Based on their similar absorption properties between 300 nanometers (nm) and 900 nm these oxyhemoglobins can be referred to as a single species.

For a healthy adult, oxyhemoglobin A can be found around 90 % although, for a baby, both oxyhemoglobin A and oxyhemoglobin F can be found.

In what follows, the oxyhemoglobins are labelled $HbO_2$.

Methemoglobin is a form of hemoglobin wherein the iron of the heme is at the oxidation state +3 ($Fe^{3+}$) and cannot link diatomic ligands of $Fe^{2+}$.

This means that the iron state +3 does not bind to $O_2$, CO and very weakly NO. However, this iron can strongly bind to ferric ligands $CN^-$ and $N_3^-$ and weakly to $H_2O$ and $OH^-$.

The methemoglobin is named metHb. Similarly to the ferrous oxy state no spectral difference is observed between metHb arising from the different human hemoglobin species.

At steady-state in presence of O2 heme (hemin) bound to serum albumin is oxidized at the oxidation state +3 ($Fe^{3+}$).

Heme bound to serum albumin can be involved in enzymatic catalysis reactions.

The heme bound to serum albumin is named heme-SA.

At steady-state in presence of O2 heme (hemin) bound to hemopexin is oxidized at the oxidation state +3 ($Fe^{3+}$).

Heme bound to hemopexin is not involved in the enzymatic catalysis reactions because of the very strong hexa-coordination of iron His-Fe-His.

The heme bound to serum hemopexin is named heme-Hx.

It can be noted that, contrary to metHb, the diatomic ligands of $Fe^{3+}$ can only bind to Heme-SA and Heme-Hx with a low value of affinity (>> mM).

The bilirubin is a degradation product of hemoglobin.

Bilirubin usually circulates in blood bound to albumin.

The biological sample 20 is preferably fluid.

The spectrophotometer 14 is adapted to measure a spectrum of a solution.

The term spectrum refers to an absorption spectrum.

The spectrum is obtained by the passing of an electromagnetic wave through the solution 20.

For instance, the electromagnetic wave is produced by a light source.

The spectrum is the evolution of the absorption of said electromagnetic wave with the wavelength.

The absorption spectrum may notably be measured by measuring the transmission of the solution at each wavelength.

The spectrum is notably obtained for wavelengths comprised between 200 nanometers (nm) and 1000 nm, preferably between 300 nm to 700 nm.

The dosing materials 16 are materials enabling to carry out a chemical analysis of at least a component of the sample 20.

According to the example of figure 1, dosing materials 16 comprises syringes 22, dosing containers 24 with perforable stopper 26.

In figure 1, only three syringes 22 and two dosing containers 24 are represented but other numbers of syringes 22 of dosing containers 24 can be consider.

The syringes 22 can be used to perforate the stopper 26 and collect the product contained in the dosing containers 24.

The content of the dosing container 24 is usually a solution of a given chemical material at a given concentration.

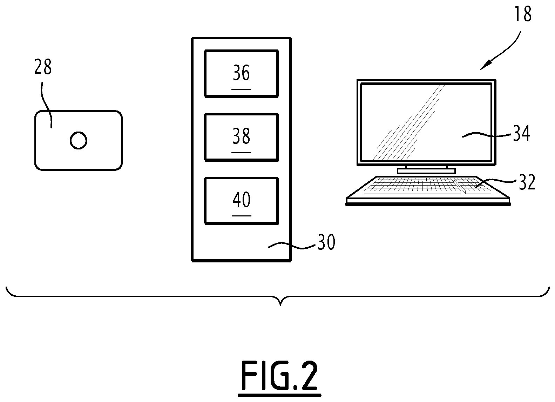

The analysis system 18 is further detailed in reference to figure 2 wherein the system 18 and a computer program product 28 are represented. The interaction between the computer program product 28 and the system 18 enables to carry out a method for determining at least one component.

System 18 is a computer. In the present case, system 18 is a laptop.

More generally, system 18 is a computer or computing system, or similar electronic computing device adapted to manipulate and/or transform data represented as physical, such as electronic, quantities within the computing system's registers and/or memories into other data similarly represented as physical quantities within the computing system's memories, registers or other such information storage, transmission or display devices.

System 18 comprises a processor 30, a keyboard 32 and a display unit 34.

The processor 30 comprises a data-processing unit 36, memories 38 and a reader 40. The reader 40 is adapted to read a computer readable medium.

The computer program product 28 comprises a computer readable medium.

The computer readable medium is a medium that can be read by the reader 40 of the processor 30. The computer readable medium is a medium suitable for storing electronic instructions, and capable of being coupled to a computer system bus.

Such computer readable storage medium is, for instance, a disk, a floppy disks, optical disks, CD-ROMs, magnetic-optical disks, read-only memories (ROMs), random access memories (RAMs) electrically programmable read-only memories (EPROMs), electrically erasable and programmable read only memories (EEPROMs), magnetic or optical cards, or any other type of media suitable for storing electronic instructions, and capable of being coupled to a computer system bus.

A computer program is stored in the computer readable storage medium. The computer program comprises one or more stored sequence of program instructions.

The computer program is loadable into the data-processing unit 36 and adapted to cause execution of the calculation steps of the determining method when the computer program is run by the data-processing unit 36.

DESCRIPTION OF THE DETERMINING METHOD

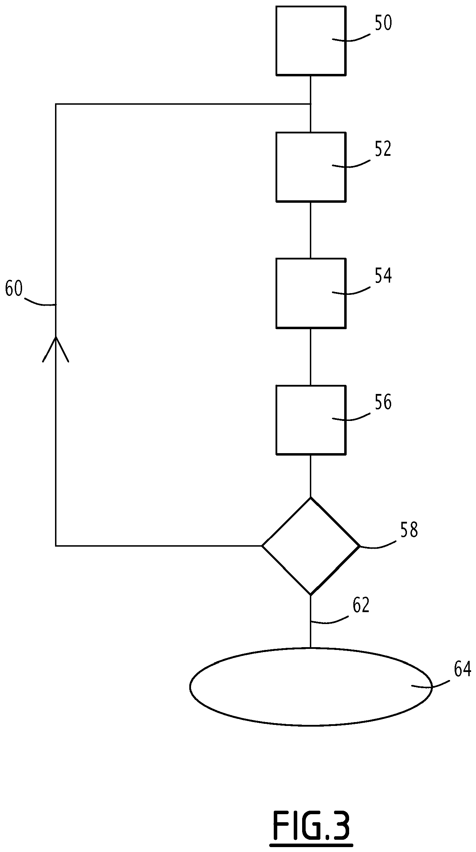

Operation of the apparatus 10 is now described by illustrating an example of carrying out the determining method as illustrated by the flowchart of figure 3.

The method comprises a step of providing named step a), a step of setting named step b), a step of iterating named step c) and a step of outputting named step d).

At the step a), an initial spectrum of the sample 20 is provided.

The spectrum is, for instance, obtained by using the spectrophotometer 14.

The spectrum can be measured just after collecting the sample 20 or after having kept the sample in conservation conditions.

For instance, the sample 20 can be kept at a temperature comprised between 5°C and 10°C during several hours (until 24 h).

Alternatively, the sample 20 can be frozen and kept at a temperature comprised between -80°C and -20°C. The freezing of the sample enables to limit the alteration of the sample with an easy stocking (freezing in liquid nitrogen before a storage at -80 °C).

The sample 20 can also be kept with a stabilization agent.

The sample 20 may also undergo fractioning, dilution or concentration. This enables to improve the detectability of the proteins in the sample.

With dilution, a sample 20 with an optical density inferior or equal to 2, preferably inferior or equal to 1.

The sample 20 can also be buffered at a pH inferior or equal to 8.

The buffering is preferably made at a pH comprised between 7.35 and 7.45, in particular equal to 7.4.

This is notably advantageous because metHb depends from the pH with a pKa of 8.

This buffering is obtained by using a buffering solution.

For instance, the buffering solution is a potassium phosphate solution or a sodium phosphate solution.

As a specific example, the buffering solution is a solution at a pH of 7.4 and consisting of 50 mM potassium phosphate and of 50 mM NaCl.

The buffering solution may be contained in a dosing container 24.

Alternatively, in another embodiment, the initial spectrum is received by the analyzing system 18, the measurement of the spectrum being carried out previously in another location and notably the location wherein the biological sample 20 is collected.

At the end of step a), an initial spectrum of the sample 20 is obtained.

At the step b), the current spectrum is set as the initial spectrum.

Step b) enables to set an initialization for the step c).

Step c) comprises the iteration of three steps.

The first step is step c1) during which the content in protein in the sample is determined.

The content in protein refers to the quantity of said protein in the sample.

The content in protein is, for instance, a mass content, a mass concentration or a molar content.

The mass content is the mass of the protein.

For instance, the mass content can be expressed in picograms (pg).

The mass concentration is the ratio of the mass of the protein and the volume of the protein in the sample.

The mass concentration can be expressed in milligrams per liter (mg/L).

The molar content is the number of moles of the protein.

The molarity content is expressed in micromoles/L ($\mu$M).

The first step c1) is carried out by using a spectral analysis of the current spectrum carried out by the spectrophotometer 14 and/or a chemical dosing using the dosing materials 16.

The second step is step c2) wherein the spectral component associated to the protein based on the determined content at the second step c2).

Since the spectral component is estimated on a restricted domain of wavelength (less than 3 or the examples below which may be larger), highly specific to the dosed protein or chemical molecule, its entire spectrum for instance on the UV/visible domain is deduced from the purified species spectrum (reference spectrum) weighted by the intensity ratio between the species signal and its reference in the measurement windows.

For instance, the formula Spectral component = ref spectrum $\times$ DO sample/DO ref (specific wavelength or domain of wavelengths can be used or simply the formula [species]) $\times$ ref spectrum normalized for instance to 1 $\mu$M.

The third step is step c3) during which the deduced spectral component is removed from the current spectrum.

Step c3) consists in subtracting to the current spectrum the deduced spectral component to obtain a new current spectrum.

Step c) is iterated successively for different proteins preferably by decreasing values of second derivatives as long as the contents of four species are not determined.

The value of second derivate of a protein is defined as the being defined as the maximum amplitude over the wavelength range of interest of the second derivative with wavelength of the spectrum of the spectral component of the protein in the sample or the maximum value over the wavelength range of interest of an absolute value of the difference between a minima and a maxima of the second derivative with wavelength of the spectrum of the spectral component of the protein in the sample.

For the sake of simplification, only the first definition is used in what follows.

This value can be measured or obtained by a simulation.

The four species are the $HbO_2$, metHb, bound heme (heme-SA or heme-Hx) and the bilirubin.

It can be noted that it can also be considered that step c) is iterated as long as the contents of five species are not determined, provided heme-SA and heme-Hx count as two species.

An example of such step c) is detailed in the next section.

At step d), the determined contents in protein are outputted.

The method is particularly adapted to analyze the various components of a sample such as a biological fluid.

The method notably provides the contents of $HbO_2$, metHb, Heme-SA, Heme-Hx and bilirubin. Other species may be outputted at step d) as will be explained in another section

## A DETAILED DESCRIPTION OF A SPECIFIC EXAMPLE OF STEP C)

In such case, the sample 20 comprises by decreasing order in second derivative: HbCO, $HbO_2$, metHb, heme-SA, heme-Hx and bilirubin. Such specific sample 2 can notably be observed for smokers (CO intoxication or CO therapies).

The determining step of each of these species will be described in what follows by providing several techniques for each species.

The goal of this analysis is to remove the easier defined species (higher amplitude, narrowest width, specific wavelength window with low or no interferences of the others spectral species). Once a species has been determined its overall spectrum is subtracted after signal normalization against its pure reference spectrum. Since $HbO_2$ and metHb are the easiest species to identify the process will start first by their determination.

### Determining content in HbCO

There is no specific technique proposed to determine the content in HbCO.

As an example, it may be considered a spectroscopic technique using the maximum peak of absorption of HbCO in the initial spectrum (preferably at 420 nm).

This enables to determine the content in HbCO in the sample 20.

The spectral content is then removed from the initial spectrum.

### Determining content in $HbO_2$

For determining the content in $HbO_2$, two kinds of techniques are proposed: spectral ones and chemical ones.

### Spectral analysis

According to this technique, a reference spectrum associated with the oxyhemoglobin is provided.

The reference spectrum is, for instance, provided with an informatic library.

The reference spectrum is obtained by a $HbO_2$ sample whose content in $HbO_2$ is superior or equal to 99 %.

Such $HbO_2$ sample can be obtained from red blood cells of a healthy non-smoker subject. The red blood cells can be washed with physiologic serum. The red blood cells undergo a lysis after a water dilution (at least 3 folds vol H2O/vol RBC). The obtained solution is centrifuged at 10000-15000 g to recover the $HbO_2$ sample in the supernatant. Traces of CO can be removed by exposure of the sample kept on ice to a light source under pure O2 to photodissociate and replace the CO by O2. Traces of metHb if present after chemical dosage can be removed by several reducing systems (for instance ferredoxin/ferredoxin reductase/NADPH) before removal by chromatography. Other alternative is the saturation the HbO2 sample under CO followed by the reduction of the metHb with a slight excess of sodium dithionite. After removal of dithionite with a desalting chromatography the CO is replaced by O2 as mentioned before.

Optionally, the $HbO_2$ sample can also be purified by a chromatographic column adapted to exclude particles with specific sizes and/or ions exchanges. HbCO spectrum can be obtained by CO gas saturation of the $HbO_2$ sample.

Then, a normalization coefficient $K_{HbO2}$ between the reference spectrum and the current spectrum is calculated.

The normalization coefficient is the ratio of the amplitude of $HbO_2$ in the sample 20 and the amplitude of the reference spectrum of the $HbO_2$.

Many definitions can be used to determine such ratio.

For instance, the normalization coefficient is defined as the minimum value of the ratio between the value of the second derivative of the $HbO_2$ and the value of second derivative of the reference spectrum.

In particular, in this example, the normalization coefficient is defined as the ratio between the value of the second derivative of the $HbO_2$ and the value of second derivative of the reference spectrum. The value of second derivative of the reference spectrum is defined as the maximum amplitude (negative sign) of the second derivative with wavelength of the spectrum.

This can be mathematically written as:

$$K = \frac{\dfrac{d^2 S_C}{d\lambda^2}}{\max\limits_{\lambda} \dfrac{d^2 S_{REF}}{d\lambda^2}}$$

Where:

- $\dfrac{d^2 A}{dB^2}$ is the operator that provides the second derivative of the quantity A over the quantity B,
- $S_C$ is the current spectrum,
- $S_{REF}$ is the reference spectrum,
- $\lambda$ designates the value of wavelengths, and
- $\max\limits_{\lambda} A$ designates maximum amplitude (negative sign) of A with wavelength.

The normalization coefficient is calculated for at least one wavelength.

The wavelength is chosen among the group consisting of 416 nanometers (nm), 543 nm and 577 nm. 577 nm is used preferentially because the signal is most weakly impacted by the spectral contribution of the other species.

In some embodiments, the normalization coefficient takes into account other coefficients.

For instance, the normalization coefficient takes into account the dilution factor of the sample during its collection; for instance the presence of an anti-coagulant.

As a specific example, the dilution factor FD is obtained based on the solute volume $V_S$, the collecting volume $V_C$ and the hematocrit ratio Htc. As an illustration, the following formula may be used:

$$FD = \frac{(1 - Htc) * V_C}{(1 - Htc) * V_C - V_S}$$

In variant or in addition, the normalization coefficient may also take into account the dilution factor of plasma in PBS.

Typically a sample is diluted into a buffered solution between 1/2 until 1/20. It depends on the signal amplitude as well as the wavelength pathway of the optical device and the volume of sample required. The goal is to minimize the amount of the biological sample for the measurement and to set the signal for all the species between 0.1 and 1 OD when possible over the entire range of analysis.

The normalization coefficient also takes into account the fact that the considered species may be bound to other molecules.

For the case of $HbO_2$, a fraction is irreversibly bound to haptoglobin which means that the evaluation of the spectrum of $HbO_2$ comprises a contribution coming from free $HbO_2$ and a contribution coming from $HbO_2$ bound to haptoglobin.

The haptoglobin can be measured by a biochemical reaction with an antibody.

The free $HbO_2$ is deduced based on the fact that the different haptoglobin isoforms have on average a binding capacity of two Hb dimers/haptoglobin. Knowing the haptoglobin concentration in g/L and taking an average MW of about 85 kDa one can estimate the binding capacity of the pool of haptoglobin in $\mu$M of $HbO_2$ based on heme and subtract this value to the total measured HbO2 for finally having a reasonable estimation of the free $HbO_2$. In case of the presence of HbCO and MetHb one may use the total hemoglobin concentration (HbCO + $HbO_2$ + MetHb) for the calculation of free $HbO_2$.

The normalization coefficient K provides access to the content in $HbO_2$.

For instance, by using the Beer-Lambert law, the following equation may be obtained:

$$c = \frac{A}{K.\xi.l}$$

Where:

- $c$ is the concentration in $HBO_2$,
- A is the absorbance of the reference sample,
- $\xi$ is the extinction coefficient of $HbO_2$, and
- $l$ is the thickness of the sample.

A, $\xi$ and $l$ are known or provided.

Notably, the extinction coefficients can be found in tables.

Thus, the above formula enables to determine the concentration which then provides access to the content in $HbO_2$.

*Chemical dosing technique*

$HbO_2$ can be dosed by adding CO.

Any method for following the reaction generated by the addition of CO can be considered.

In the context of this example, a spectroscopic follow-up can be advantageous in so far as the apparatus 10 is already provided with a spectrophotometer 14.

In case of a chemical dosage values may arise from a maxima or minima of the variation of absorption after a chemical reaction or preferably the absolute value of the difference between a minima and a maxima. Those later values may also arise from the second derivative of the variation of the absorption after the chemical reaction.

Determining content in metHb

Similar remarks made previously for the spectral analysis of the oxyhemoglobin apply for the methemoglobin by only replacing oxyhemoglobin by methemoglobin. These remarks are not repeated in what follows. Only the specific differences are highlighted.

The wavelength used for calculating the normalization coefficient is chosen in the range of 350 nm to 700 nm.

Methemoglobin is dosed by adding KCN.

The dosing can be achieved by using a solution of KCN whose concentration in anion $CN^-$ is comprised between 100 $\mu$M and 500 $\mu$M, preferably between 150 $\mu$M and 250 $\mu$M, more preferably equal to 200 $\mu$M.

The incubation time is, for instance, of 5 minutes. Indeed, the bimolecular rate for $CN^-$ binding to metHb is about 100 /M/s at room temperature which predicts using 200 $\mu$M of KCN over 99 % of binding after a few minutes. The same applies if NaCN is used instead of KCN, notably in terms of concentration

As metHb is also reactive with anions $N_3^-$, $NaN_3$ can be used instead of KCN to dose metHb.

In such case, the dosing can be achieved by using a solution of $NaN_3$ whose concentration in anion $N_3^-$ is comprised between 500 $\mu$M and 1000 $\mu$M, preferably more preferably equal to 500 $\mu$M.

As previously, the dosing may be followed using a spectroscopic technique.

Determining content in heme-SA

For determining the content in heme-SA, two kinds of techniques are proposed: spectral ones and chemical ones.

*Spectral analysis of the current spectrum*

Similar remarks made previously for the spectral analysis of $HbO_2$ apply for the heme-SA by only replacing $HbO_2$ by heme-SA. These remarks are not repeated in what follows. Only the specific differences are highlighted.

The reference spectrum is obtained by a heme-SA sample using an excess of human albumin vs heme. A spectrum of human albumin is measured before and after addition of heme after waiting the binding reaction achievement. The heme-SA spectrum is obtained after subtracting the initial free albumin spectrum.

For instance, such heme-SA sample may be obtained as follows. Heme can be dissolved in a NaOH solution 0.1 N and kept deprived of sun before being added to an AS solution filtrated on 0.2 $\mu$M with a final solution with 1 mole heme for 5 moles SA. After 20 minutes of equilibration without light, the solution is obtained.

Optionally, the solution can be buffered as described at step a).

The wavelength used for calculating the normalization coefficient is chosen in the range of 200 nm to 800 nm.

Preferably, the range may be comprised between 300 nm and 700 nm, between 350 and 460 nm and/or 600 and 700 nm. In the red part of the spectrum a first estimation can be made to constrain the other analysis in the purple part. When heme-SA is concentrated the second derivative can give an estimation of its concentration. At lower concentration the signal can be simulated by subtracting a certain amount of the reference spectrum to get the best simulation of a straight line passing through two points chosen between 610 and 600 nm and 675 and 700 nm respectively; the latter point being constrained to belong to the measured spectrum.

*Chemical dosing technique*

Heme-SA can be dosed by adding sodium dithionite (DTN) under CO. The reference spectra for the variation of absorption arising from the reduction by DTN upon CO: heme($Fe^{3+}$)-SA -> heme($Fe^{2+}$-CO)-SA is obtained from a patient blood depleted in Hx with plasma heme. The spectrum is slightly different from that obtained with purified human SA in PBS due to the plasma biochemical conditions and binding interaction state.

Determining content in heme-Hx

As for heme-SA, determining the content in heme-Hx may use two kinds of techniques: with or without chemical addition. This also depends on the amount of heme present in the biological fluid and the binding competition between AS and Hx. Except for heme based treatment, natural high amount of heme in plasma is correlated to a low heme-Hx amount after its removal from the circulation for heme degradation. Only remains heme-SA simply because Hx concentration is about 50 less than that of AS and the Hx turnover not enough efficient. Heme competition for binding between SA and Hx is then only observed at low heme concentration. In other words, the detection in vivo of heme-Hx is linked to the concomitant detection of heme-SA if heme-SA component cannot be removed after a separate determination. Consequently heme-SA determination at low heme concentration may involve similar operations. The determination of both species requires a simulation, preferably between 350 and 460 nm, using a linear combination of their respective second derivative spectrum.

*Spectral analysis*

Similar remarks made previously for the spectral analysis of the $HbO_2$ apply for heme-Hx by only replacing $HbO_2$ by heme-Hx. These remarks are not repeated in what follows. Only the specific differences are highlighted.

The wavelength used for calculating the normalization coefficient is chosen in the range of 300 nm to 700 nm depending on the used techniques.

The reference spectrum is obtained by a heme-Hx sample using a slight excess (20 %) of human hemopexin vs heme in a similar way developed for heme-SA.

*Chemical dosing techniques*

Heme-Hx can be dosed by adding sodium dithionite (DTN) under CO. The reference spectra for the variation of absorption arising from the reduction by DTN upon CO: heme($Fe^{3+}$)-Hx -> heme($Fe^{2+}$-CO)-Hx is obtained with purified human Hx in PBS as mentioned above. In plasma Heme-Hx is determined by simulation of the variation of absorption upon reduction with both Heme-Hx and Heme-SA reference spectra after subtraction of the contribution of the metHb reduction estimated apart by chemical dosage.

In variant, heme-Hx can be dosed by adding sodium dithionite (DTN). This dosage is a strong tool since it does not require a calibration curve as for other typical dosage and is based on the plasma Hx pool binding capacity. The reference spectra for the variation of absorption arising from the reduction by DTN in absence of oxygen consumed by the reagent: heme($Fe^{3+}$)-Hx -> heme($Fe^{2+}$)-Hx is obtained again with purified human Hx in PBS. The heme($Fe^{3+}$)-Hx sample is deoxygenated under $N_2$ or Ar before addition of DTN (catalase is added to prevent oxidative side reactions). Only this technique allows a direct measurement of Heme-Hx content between 520 and 600 nm. Indeed in the deoxy state Hx exhibits two intense and narrow absorption peaks which can be detected using the second ($2^{nd}$) derivate whereas the other deoxy species ($HbFe^{2+}$, $hemeFe^{2+}$-SA, bilirubin) exhibit a broad peaks. The Heme-Hx is estimated at the maximum amplitude of second derivative for the plasma supplemented with heme which is located at 561 nm or preferably using the absolute value of the difference between the maxima (550-551 nm) and minima (561 nm) of the second derivative. Note that the small contributions to the second derivative spectra for the other species at both wavelengths are equivalent in amplitude and sign so that they do not influence the heme-Hx measurement after subtraction. Finally a global simulation may be used between 540 and 575 nm.

According to another example, finally total Hx content can be dosed by adding heme in the biological sample. A slight excess of heme with regard to the upper standard limit of its biological concentration is added at pH between 6.5 and 7.4, preferentially pH 7.0 for incubation about 30 mn at room temperature before the Hx measurement after heme binding. As such this dosage allows the determination of Hx content in a biological fluid in molarity (heme binding capacity). Typically 20 $\mu$M of heme is added for Hx dosage in plasma after setting the pH by dilution with a buffer (i.e. ½ dilution). When heme concentration in the plasma is elevated heme addition may not be necessary since Hx is obviously already saturated. Knowing that Hx owns only one site of heme binding, the Hx concentration is converted in g/L using an average molecular weight of 60 kDa.

Interestingly, since there is an inversed correlation between plasma heme and Hx in absence of up-regulation for instance during certain inflammation conditions, the measurement of Hx gives an indirect status of the heme concentration and vice et versa. This means that an abnormal high concentration of Hx with low amount of heme may indicate a specific physio-pathological symptom. Low Hx concentration may be due to the presence of an intra-vascular hemolysis with Hb degradation and/or due to a dyserythropoiesis in bone marrow. Down-regulation is also possible in case of a strong iron overload.

Determining content in bilirubin

For determining the content in bilirubin, only a spectral analysis is proposed.

Similar remarks made previously for the spectral analysis of the $HbO_2$ apply for the bilirubin by only replacing $HbO_2$ by bilirubin. These remarks are not repeated in what follows. Only the specific differences are highlighted.

The reference spectrum is obtained by a bilirubin sample whose content in bilirubin is about 10 $\mu$M.

Such bilirubin sample is a sample of bilirubin with an excess of serum albumin (SA).

For instance, such bilirubin sample may be obtained as follows. Bilirubin can be dissolved in a NaOH solution 0.1 N and kept deprived of sun before being added to an AS solution filtrated on 0.2 $\mu$M with a final solution with 1 mole bilirubin for 2 moles of SA. After 20 minutes of equilibration without light, the solution is obtained. Other alternative is the use of a plasma sample with about 50 $\mu$M of bilirubin of a blood donor without a chronic pathology (different ratio of bilirubin conjugated with glucuronic acid or unconjugated can be used). The bilirubin spectrum is obtained after subtraction of the remaining $HbO_2$ component (absence of other Hb or degradation products should be assed).

The solution is buffered in PBS preferably at pH 7.4 as described at step a).

The wavelength used for calculating the normalization coefficient is chosen in the range of 350 nm to 700 nm.

Preferably, the wavelength used for calculating the normalization coefficient is chosen in the range of 440 nm to 540 nm. More preferably, the wavelength used is around 501 nm.

Graphical illustrations

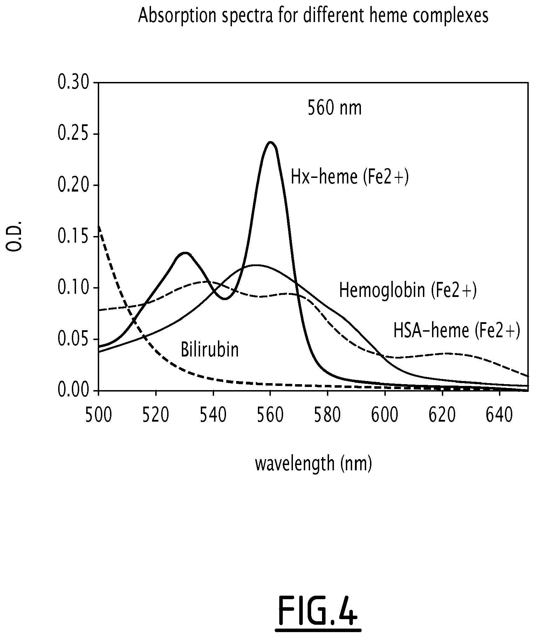

Figures 4 to 6 illustrate graphically some steps of the previous determining of contents in a specific case of determining the content in hemopexin.

Figure 4 shows spectra of different species reduced in the absence of $O_2$. The spectrum of $HxFe^{2+}$ will be very predominant compared to the other ones in the second derivative since it exhibits two very thin bands.

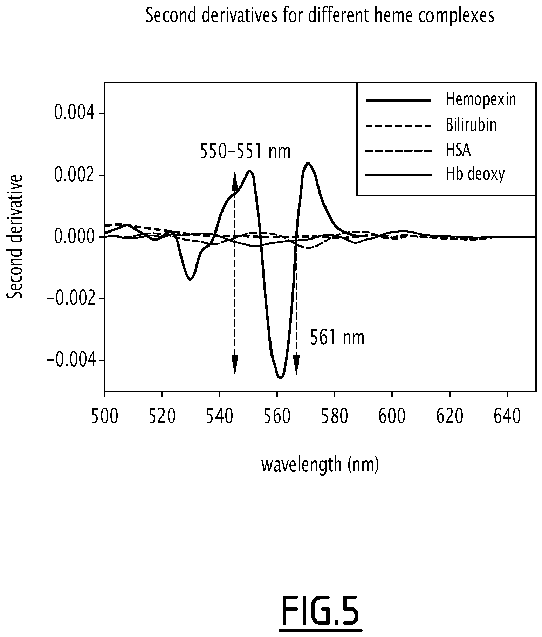

Figure 5 illustrates a way to circumvent such problem for hemopexin. It represents the analysis of the second derivative either at 561 nm (first arrow in dotted lines) or by taking the difference between the maximum (around 550-551 nm in reference to a second arrow in dotted lines) and the minimum at 561 nm. This enables to eliminate the weak contribution of the other species which does not vary too much between 550-551 nm and 561 nm. The two analyses may be used.

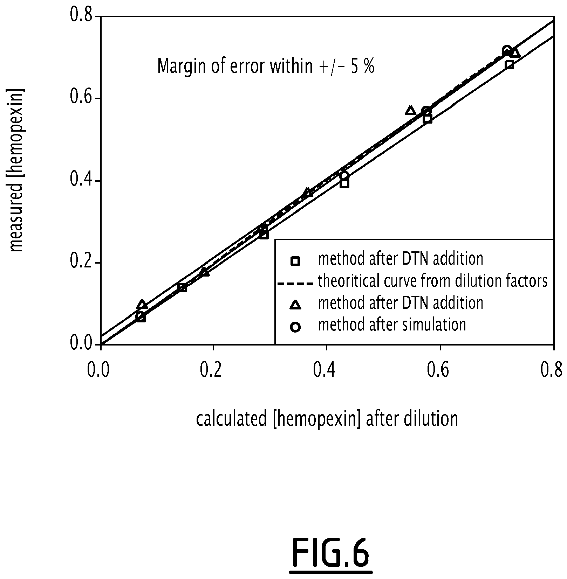

Figure 6 illustrates a calibrating graph using the measurement of heme. The precision can be as low as 0.1 g/L and even below 0.05 g/L.

OTHER ELEMENTS

The method that has been described previously can be extended to many other species.
It can notably be cited:

- carboxylated hemoglobin (HbCO),
- ferryl hemoglobin,
- plasma heme which can notably dosed by adding CO and/or sodium dithionite. The iron atom of heme $Fe^{3+}$ is reduced in presence of sodium dithionite and then bound to CO in absence of $O_2$ (the $O_2$ reacts with sodium dithionite). This renders a spectral determination possible,
- myoglobin,
- porphyrin,
- porphobilinogen,
- urobilin,
- products of catabolism of heme by heme oxygenase,
- products of bilirubin degradation/oxidation: biliverdin, heme boxes,
- products of organ dysfunction and cytolysis (coenzyme molecules, heme proteins, cytochrome P450, transaminases), and
- metabolic substrates, metabolic degradation.

*Alternative Chemical Method to Determination of Hb, Heme and Hb Degradation Products in Blood Plasma and Other Fluids / Biological Specimens*

A similar formalism is used. "Reference spectra" or "SR" are absorption spectra obtained for a single protein. The reference spectra are generally obtained before implementing the method of the invention. Differential reference spectra "SDR" correspond to changes in absorbance associated with a chemical reaction for a given species.
"Calculated spectra" or "calculated intermediate spectra" are spectra obtained after subtraction from the measured spectral species SA, from its reference spectrum weighted by a "K" normalization factor, ratio between the ratio of the signal amplitude of the determined species and its reference.
This method is based on the reduction of plasma $Fe^{3+}$ heme by sodium dithionite in the presence of CO. It involves the addition of two additional chemical reactions compared to the previously described method for which only the reaction in the presence of $CN^-$ was used. The iron atom of $Fe^{3+}$ heme plasma species is reduced in the presence of sodium dithionite for a previously equilibrated sample under CO in the absence of $O_2$ (itself consumed by dithionite). The first step is to replace the $O_2$ reduced globes $Fe^{2+}$ in air with CO (affinity of CO 200 times higher than for $O_2$). Once the bond $Fe^{2+}$-CO formed, it is considered irreversible without modification of the chemical properties of iron after addition of the chemical reagents necessary for the assay and therefore without any change in the spectral component associated with it. After addition of CO only species with $Fe^{3+}$ remain sensitive to the dosing method.
The order of chemical reactions is as follows:

1) addition of KCN after recording of the plasma spectrum SA as previously described
2) addition of gaseous CO
3) addition of sodium dithionite.

However, it should be noted that steps 1) and 2) can be reversed without changing the final result of the analysis.
The different steps of the measurement protocol are as follows:

1) SA spectrum of plasma under air buffered at pH 7.4 +/- 0.1. The dilution factor will be taken into account for the calculation of heminised species.
2) $SA^{CO}$ plasma spectrum after addition of CO: chemical dosing of $HbFe^{2+}$ bound to $O_2$ in air, free and / or complexed with haptoglobin (the sentence is removed since the calculation of free Hb is given more haptoglobin) from the spectral change induced by the replacement of $Fe^{2+}$ bound to $O^2$ by CO (SA - $SA^{CO}$).
The concentration of CO is between 100 $\mu$M and 1 mM (solubility of gaseous CO at 25 ° C is about 1 mM under 1 atm) after addition of CO gas or a known volume of PBS saturated with CO. The incubation time is about 1 minute.
3) $SA^{CO+KCN}$ spectrum of the plasma in the presence of CO after addition of KCN: chemical determination of the already described $Fe^{3+}$ globin previously called metHb from the spectral change induced by the binding of $CN^-$ to

$Fe^{3+}$ ($SA^{CO}$ - $SA^{CO+KCN}$) . It is conceivable to use also other chemical compounds for NaCN or even $NaN_3$ et $KN_3$ dosing ($N_3^-$ being a ligand of $Fe^{3+}$). The concentration of the cyanide salts is about 0.2 mM, between 0.5 and 1 mM for that azide salts. The incubation time is between 3 minutes and 5 minutes. It should be noted that the affinities of heme bound to HSA and Hx are too weak to give a binding signal for ferric ligands in this concentration range.

4) $SA^{CO+KCN+DTN}$ plasma spectrum in the presence of CO and KCN after addition of sodium dithionite: chemical assay of serum $Fe^{3+}$ plasma heme bound to human albumin (HSA) and serum hemopexin (Hx) from spectral change induced by $Fe^{3+}$ reduction ($SA^{CO+KCN}$- $SA^{CO+KCN+DTN}$). Once reduced $Fe^{2+}$ binds to the CO present in solution. The signal will be composed of several spectral transitions, namely:

- HSA-heme ($Fe^{3+}$) -> HSA-heme ($Fe^{2+}$)-CO,
- Hx-heme ($Fe^{3+}$) -> Hx-heme ($Fe^{2+}$)-CO and

metHb ($Fe^{3+}$) - $CN^-$-> Hb ($Fe^{2+}$)-CO which will be calculated from the previous assay (3) and reference spectra of the species involved.

If the metHb dosing is performed separately on another sample then the spectral transition will be metHb ($Fe^{3+}$) -> Hb ($Fe^{2+}$)-CO in the absence of $CN^-$ or $N_3^-$. All the dosings (will preferably be performed at physiological pH 7.4 +/- 0.1 as well as for the reference spectra. The incubation time is about 20-30 min after addition of dithionite at a concentration of about 0.5-1 mM. Finally, the calculated signal, based on the spectral variation induced by the addition of dithionite to which the contribution to the signal of the reduction of the metHb under CO is subtracted, will be analyzed as a linear combination of the reference spectra for the spectral contributions of HSA-heme ($Fe^{3+}$) and Hx-heme ($Fe^{3+}$) after reduction in the presence of CO. The total plasma heme will be calculated as the sum of the concentrations of HSA-heme ($Fe^{3+}$) and Hx-heme ($Fe^{3+}$). Only HSA-related heme is likely to interact with other molecular targets or dissociate to tissues. Since certain anticoagulants rise the pH of plasma (heparin or EDTA) and could change the heme distribution among the scavenger species, plasma under citrate or simply serum should give a closer status of the heme fate (no change of the total heme calculation). As for heme-SA, the determination of heme-Hx is based on a purely spectral analysis developed above or after chemical dosing developed in this section. The concentration of heme-Hx will be inversely proportional to the total plasma heme due to the low turnover of Hx after hepatic endocytosis of its heme complexed form.

The differential reference spectrum of Hx after CO reduction at pH 7.4 +/- 0.1 is obtained from the lyophilized protein (Sigma Aldrich). The Hx / heme ratio is about 1.2, ie an excess of protein in the presence of catalase and superoxide dismutase. The Hx-heme spectrum ($Fe^{3+}$) is then recorded. After deoxygenation of the sample and equilibration under CO, the Hx-heme ($Fe^{2+}$)-CO spectrum is finally measured after reduction of iron in the presence of approximately 200 $\mu$M of sodium dithionite a few minutes after addition. Normalization is based on the extinction coefficient of Hx-heme ($Fe^{3+}$) at 413-414 nm and should be comparable to that obtained after calculation of dilution of heme stock in NaOH in the measuring vessel; the concentration of the heme stock is estimated by weighing or dilution in an HSA solution (HSA ratio / heme> 2) based on the heme-HAS extinction coefficient at 403-404 nm (second derivative as well).

The differential reference spectrum of HSA after reduction under CO (20-30 min of incubation) is obtained from sickle cell patient blood with an excess of heme (approximately 10 $\mu$M). Since Hx depletion is almost total (confirmation by dosing technique), the spectral component of the plasma heme is derived from HSA-heme ($Fe^{3+}$) after subtraction of the low metHb fraction. The differential reference spectrum is obtained after dilution of the plasma in PBS pH 7.4 +/- 0.1 using the second derivative normalized from HSA-heme ($Fe^{3+}$) in violet between 350 nm and 460 nm. The differential reference spectrum of plasma heme-HSA after reduction under CO is slightly different from that obtained from purified human albumin in PBS due to biochemical conditions and conformation of the binding site in the plasma (presence of bilirubin). At high [heme] (depletion in heme-Hx) only the differential spectrum from HSA can be used for the simulation of the signal in the violet or/and in the visible pre.

The second derivative representation of the differential spectrum, when measurable (depending on signal to noise ratio), may be also used to estimate heme binding between HSA and Hx between 350 and 450 nm. Note that the normalized maximum amplitude (negative sign between 422-424 nm) of the heme-HSA and heme-Hx second derivatives are close so that their average value can be used to estimate directly the total plasma heme concentration with an accuracy of about +/- 5 % (using the differential spectrum the accuracy the accurate is about +/- 10 %).

At high [heme] (depletion in heme-Hx) only the seconde derivative of the differential spectrum from HSA can be used for the simulation of the signal in the violet or/and in the visible preferably between 520 and 620 nm.

At this stage of the analysis, the contribution of the various species chemically determined: HbO2, MetHb, HSA-heme, Hx-heme (reference spectra obtained under air) can be subtracted from the initial spectrum of the plasma SA so after signal processing to analyze other spectral species as per example total bilirubin (free and conjugated).

The analytical equations used for the different assays are as follows (in bold the normalization coefficients in relation to the different dosages)

1) Determination of HbFe2 + initially bound to $O_2$ by addition of CO:

$$SA\text{-}SA^{CO} = K^{CO} \times SDR^{CO}\ (SDR^{CO} = SR^{HbO2} - SR^{HbCO})$$

2) MetHbFe$^{3+}$ dosing by addition of KCN:

$$SA^{CO} - SA^{CO+KCN} = K^{KCN} \times SDR^{KCN}\ (SDR^{KCN} = SR^{MetHb} - SR^{MetHb\text{-}CN})$$

3) HSA and Hx-related plasma heme assay by addition of DTN:

- $SA^{CO+kcn+DTN} - SA^{CO+kcn} = K^{HSA\text{-}heme} \times SDR^{HSA\text{-}hemeFe2+CO/HSA\text{-}hemeFe3+} + K^{Hx\text{-}heme} \times SDR^{Hx\text{-}hemeFe2+CO/Hx\text{-}hemeFe3+} - K^{KCN} \times SDR^{HbCO/\ metHbCN}$ ($K^{KCN}$ calculated above)

wherein :

- $SDR^{HbCO/\ metHbCN} = SR^{HbCO} - SR^{MetHb\text{-}CN}$
- $SDR^{HSA\text{-}hemeFe2+CO/HSA\text{-}hemeFe3+} = SR^{HSA\text{-}hemeFe2+CO} - SR^{HSA\text{-}hemeFe3+}$
- $SDR^{Hx\text{-}hemeFe2+CO/Hx\text{-}hemeFe3+} = SR^{Hx\text{-}hemeFe2+CO} - SR^{Hx\text{-}hemeFe3+}$

4) HSA and Hx-related plasma heme assay by addition of DTN:

$$SC^{CO+kcn+DTN} = SA - K^{CO} \times SR^{HbO2} - K^{KCN} \times SR^{MetHb} - K^{HSA\text{-}heme} \times SR^{HSA\text{-}hemeFe3+} - K^{Hx\text{-}heme} \times SR^{Hx\text{-}hemeFe3+}$$

The SC spectrum will be analyzed for the determination of other spectral species such as HbCO and bilirubin.

Data analysis may involve mathematical processing using the representation of the second derivative to amplify the best resolved absorbance peaks; this is the case in this chemical assay for the CO-ligated species which have a very intense absorbance band and a narrow width at 420 nm. The field of analysis covers the entire spectrum of white light and near UV between 300 and 700 nm more particularly between 350 nm and 450 nm where the absorbance bands are the most intense for heminized species. This method can also be used in humans for the determination of heme / Hb in other body fluids, cell and tissue extracts from biopsy. Its field of application can be extended to dosages in animals and for cell culture.

The quantification of the heme uses, for example, the use of a PBS buffer preferably at pH 7.4 containing human HSA or of animal origin between 100 and 500 $\mu$M (whose reference spectra in the presence of heme will be measured and standardized)

*Hemopexin determination methods by UV / visible spectroscopy*

The methods are based on the same analytical and mathematical approaches previously developed. They are based on the addition of the Hx substrate, in this case heme in the plasma or in any other biological medium in which it is to be assayed. It will be developed in this text the dosage in the plasma.

The first method is to measure a differential spectrum after adding heme. The absorbance spectrum of the assay medium ($SA^{baseline}$) will be measured alone and in the presence of an excess of heme. The differential spectrum $SN^{hème} - SA^{baseline}$ will then be calculated taking into account the dilution factor in order to analyze the signal resulting from the binding of heme to the serum proteins HSA and Hx. The second derivative of the signal will be modeled as a linear combination of the second derivatives of the two major protein complexes at $\lambda$ belonging to [350 nm, 700 nm], preferably at $\lambda$ belonging to [350 nm, 460 nm]. The high intensity of the second derivative of the Hx-heme spectrum compared to that of HSA-heme makes the method very sensitive to the determination of Hx even in excess of binding of heme to HSA.

However, because of the subtraction of the absorbance of the medium to be analyzed which may have a heme-bound fraction of heme in the stationary state, particularly for plasmas with low intravascular hemolysis (Hx is depleted in case of chronic moderate haemolysis), it is desirable to measure beforehand the fraction of heme-Hx before adding heme by the methods described above in order to calculate the total of heme-Hx before and after addition of heme.

The second method consists in measuring the spectrum of a plasma in the same way with an excess of heme but after adding sodium dithionite in order to reduce the heme and to obtain the Hx(Fe$^{2+}$)-heme spectrum. The

Hx(Fe$^{2+}$)-heme spectrum is obtained as described above after addition of heme to lyophilized Hx and addition of dithionite after deoxygenation under N$_2$ (addition of SOD and catalase). Indeed, this species is associated with a spectrum that has two absorbance peaks between 500 nm and 600 nm, one of which is thin and intense at 560 nm. As a result, the second derivative of the plasma sample after addition of sodium dithionite is very sensitive to the presence of Hx(Fe$^{2+}$)-heme while the spectral contributions of the other species present l'HbFe$^{2+}$, l'HSA(Fe$^{2+}$)-heme and bilirubin will be usually negligible in this range of wavelengths (broad spectra and lower intensity). Nevertheless in case of a low amount of Hx other components measured from Hb and bilirubin dosages as well as the HSA-heme component based on the known amount of heme added can be removed to the signal after dithionite addition for a more easily measurement of the Hx content. The latter method relies only on the acquisition of a single SA$^{heme+dithionite}$. The analysis is developed in more details bellow. Finally using the same methodology for the determination of Hx(Fe$^{2+}$)-heme species, the dosage of the total plasma heme could be achieved by addition of purified free Hx after plasma dilution in the same buffer conditions described below to displace the heme binding steady-state in interaction with the serum components toward Hx its strongest scavenger.

Both plasma hemopexin dosing methods rely on the addition of heme to saturate the Hx to be dosed. The incubation pH is an important parameter to control. The incubation pH is between 6.5 and 7.4, in particular equal to 7.0, to decrease the heme affinity for HSA and thus promote Hx binding. The pH of the plasma being greater than 7.4 with the anticoagulants heparin and EDTA unlike the citrate closer to physiological pH or serum the samples will be diluted to ½ in a 50 mM PBS 50 mM phosphate NaCl pH 6.0 for samples with pH> 7.4 and in the same pH 6.5 buffer for samples with pH around 7.4. The final pH of incubation is close to 7.0. It should be noted that other buffers could be used to buffer the sample at a pH of 7.0. The heme is then gradually added with stirring so as to obtain a final concentration of about 20 μM from a stock solution of heme of 1 mM in 0.1N NaOH prepared on the day of the measurement (for example Sigma Aldrich). If for a pathology the concentration of hemopexin is abnormally high (> 1.2 g / L) the excess of heme may then be increased. The concentration of the stock is determined by weighing and possibly controlled by UV / visible spectrophotometry after dilution in a PBS buffer of pH 7.4 containing HSA in excess 20 min after dilution.

The incubation of the plasma sample in the presence of hemin lasts 30 minutes and then the sample is diluted 1/3 in a pH 7.4 PBS for the final measurement, ie the SA$^{heme}$ spectrum for the first method and / or the spectrum SA$^{heme+dithionite}$ for the second chemical method after addition of dithionite; the same sample can therefore be used for both types of Hx assays. If there is a significant difference in measured Hx $\geq$ 0.05 g / L by the chemical method in the presence of dithionite, this indicates the presence in the plasma after removal of Hx-related plasma heme. It should be noted that a plasma aliquot can be diluted in the same way without addition of hemin to measure the reference spectrum (SA$^{baseline}$) used in the first dosing method and then used for the characterization of the other biomarkers of the hemolysis.

The sodium dithionite solution is prepared in a clogged tube with low permeability to gas previously deoxygenated with nitrogen. The reagent is then dissolved in a PBS of pH 7.4, deoxygenated under N$_2$, optionally after degassing and usable for 24 hours at a concentration of approximately 0.1 M. In the absence of nitrogen, the dithionite can be prepared with a buffer degassed or not degassed, however, in this case the tube containing the dithionite must be completely filled with buffer to remove the entire gas phase containing reactive species: O2 and dithionite (the dithionite is removed without stirring the solution for limiting any remaining gas phase diffusion toward the liquid phase). It is also preferable to deoxygenate the plasma + heme sample or to eliminate the gaseous phase in the measuring cell before adding dithionite to a final concentration of preferably 0.5 mM. Similarly, it is important to avoid vigorously equilibrating the plasma after addition of dithionite in the presence of a gaseous phase containing O$_2$, since the dithionite is consumed by reaction with the O$_2$ contained in the gaseous phase. After addition of dithionite the spectrum is measured without delay preferably within one minute. The concentration of Hx is measured using the second derivative by simulation of the second derivative between 540 nm and 580 nm, taking its minimum at 561 nm or preferably the difference between the minimum at 561 nm and the maximum at 551 nm with respect to a reference spectrum Hx(Fe$^{2+}$)-heme obtained from purified Hx and heme.

The main advantage of these methods is that the analysis is based on reference spectra. It is therefore not necessary to make a calibration straight line (unlike an Elisa test). These dosing methods measure plasma Hx binding capacity for heme and are not influenced by Hx glycosylation variations or the presence of molecular aggregates as for turbidimetric methods after precipitation with antibodies. The final result of the dosing method is expressed in μM of Hx and can be converted to g/L of Hx taking an average molecular weight of 60 kD.

APPLICATIONS

Many applications of this determining method can be considered. Some of them are developed in this section.

Are notably developed the applications linked to heme-related or hemoprotein-related disorder and notably the disease involving a heme-related or hemoprotein-related disorder at least as a symptom. Heme-related or hemoprotein-related disorders notably encompass hemoglobin-related diseases or RBC diseases. As another specific example, heme-related or hemoprotein-related disorders notably encompass disorders of the intravascular hemolysis.

However, heme-related or hemoprotein-related disorder does not encompass all disease related to blood. Notably, cerebral aneurysm, such as subarachnoid hemorrhage, is not a heme-related or hemoprotein-related disorder.
When relevant, the applications are in vitro applications.

*Method for predicting*

For this application, it is proposed a method for predicting that a subject is at risk of suffering from a heme-related or hemoprotein-related disorder.
The method for predicting comprises a step for carrying out the steps of the method for determining at least one content in protein in a biological sample of the subject or its metabolized/degradation products (natural pigments as in the other below applications), to obtain determined parameters.
The method for predicting also comprises a step of predicting that the subject is at risk of suffering from the heme-related or hemoprotein-related disorder based on the determined parameters.

*Method for diagnosing*

This application corresponds to a method for diagnosing a heme-related or hemoprotein-related disorder.
The method for diagnosing comprises carrying out the steps of the method for determining at least one content in protein in a biological sample of the subject, to obtain determined contents in protein.
The method for diagnosing also comprises carrying out a step of diagnosing heme-related or hemoprotein-related disorder based on the determined contents in protein.

*Method for treating*

This application corresponds to a method for treating a heme-related or hemoprotein-related disorder (follow-up).
The method for treating comprises carrying out the steps of the method for determining at least one content in protein in a biological sample of the subject, to obtain determined contents in protein (i.e. steady-state comparison of at least one content molecule before and after treatment).
The method for treating also comprises administrating a medicine (bone marrow transplantation, gene therapy) treating the heme-related or hemoprotein-related disorder based on the determined contents in protein.

*Method for defining stages*

In this application, it is proposed a method for defining stages of a heme-related or hemoprotein-related disorder.
The stages of a disease are the different levels of a disease. The definition of the stages are usually defined based on the symptoms of the disease.
The method for defining comprises carrying out the steps of a method for determining at least one content in protein in a biological sample of the subject, to obtain determined contents in protein.
The method for defining then comprises defining the stages of the heme-related or hemoprotein-related disorder based on the determined contents in protein as well as in association with other biological parameters (predictive algorithm for a diagnosis).

*Method for identifying a target*

This application corresponds to a method for identifying a therapeutic target for preventing and/or treating a heme-related or hemoprotein-related disorder.
The method for identifying comprises carrying out the steps of the method for determining at least one content in protein in a biological sample of images of a first subject, the first subject being a subject suffering from the heme-related or hemoprotein-related disorder.
The method for identifying also comprises carrying out the steps of the method for determining at least one content in protein in a biological sample of a second subject, to obtain second determined contents in protein, the second subject being a subject not suffering from the heme-related or hemoprotein-related disorder.
The method for identifying further comprises a step of selecting a therapeutic target based on the comparison of the first determined contents in protein and the second determined contents in protein.

*Method for identifying a biomarker*

In this application, a method for identifying a biomarker is proposed.

The biomarker can be one biomarker among a diagnostic biomarker of a heme-related or hemoprotein-related disorder, a susceptibility biomarker of a heme-related or hemoprotein-related disorder, a prognostic biomarker of a heme-related or hemoprotein-related disorder or a predictive biomarker in response to the treatment of a heme-related or hemoprotein-related disorder.

The method for identifying comprises carrying out the steps of the method for determining at least one content in protein in a biological sample of a first subject, to obtain first determined contents, the first subject being a subject suffering from the heme-related or hemoprotein-related disorder.

The method for identifying comprises carrying out the steps of the method for determining at least one content in protein in a biological sample of images of a second subject, to obtain second determined contents in protein, the second subject being a subject not suffering from the heme-related or hemoprotein-related disorder.

The method for identifying comprises selecting a biomarker based on the comparison of the first determined contents in protein and the second determined contents in protein.

*Method for screening a compound*

This application corresponds to a method for screening a compound.

The compound is a medicine.

The medicine has an effect on a known therapeutical target, for preventing and/or treating heme-related or hemoprotein-related disorder.

The method for screening comprises carrying out the steps of the method for determining at least one content in protein in a biological sample of a first subject, to obtain first determined contents in protein, the first subject being a subject suffering from the heme-related or hemoprotein-related disorder and having received the compound.

In this context, the term "receive" encompasses any ways of administration of the medicine.

The method for screening also comprises carrying out the steps of the method for determining at least one content in protein in a biological sample of a second subject, to obtain second determined contents in protein, the second subject being a subject suffering from the heme-related or hemoprotein-related disorder and not having received the compound.

The method for screening further comprises selecting a compound based on the comparison of the first determined contents in protein and second determined contents in protein.

*Method for qualifying or disqualifying*

This application corresponds to a method for qualifying and disqualifying blood bags. Blood is a generic terms encompassing red cells and plasma.

The bags are containers.

The bags contain a biological sample of subject.

The method for qualifying or disqualifying comprises carrying out the steps of the method for determining at least one content in protein in the medical bag, to obtain determined contents in protein.

The method for qualifying or disqualifying comprises also comprises qualifying or disqualifying medical bags based on the determined contents in protein.

For instance, in case, the contents of protein show that the quality of the sample is so degraded that the sample cannot be used anymore, the bag is disqualified.

The idea of such specific application to analyze the content of hemolysis in the preservation medium but also if there is any doubt to collect red blood cells to incubate in an isotonic pb at pH 7.4 (the pH of the bags decreases). with the preservation time and is usually between 6 and 7) in the presence of 100-500 $\mu$M albumin (in vivo) to recover everything that could interact with the membrane and also to measure the most fragile component that will lowering the transfusion yield. For plasma bags to detect the presence of an abnormal hemolysis content based on heme and its derived species but also to estimate the total Hx concentration which can be useful as a therapeutic agent for treating a patient with an ongoing hemolysis by plasma exchange. (haptoglobin can be measured by addition of Hb before deoxygenation and reduction with dithionite in presence of catalase; indeed spectra of deoxy dimer bound to haptoglobin and deoxy Hb tetramer are different so that an addition of Hb equivalent to the upper limit of normal haptoglobin range allows the estimation of the protein using the same methodology developed in this document). Hence this application is equivalent to a quality control for therapeutic applications

*Method for monitoring a treatment*

This application corresponds to a method for monitoring a treatment against heme-related or hemoprotein-related disorder in a subject suffering from the hemoglobin related disease and having received the treatment.

The method for monitoring comprises carrying out the steps of the method for determining at least one content in protein in a biological sample of the subject, to obtain determined contents in protein.

The method for monitoring further comprises monitoring the determined contents in protein with monitoring a treatment against heme-related or hemoprotein-related disorder in a subject suffering from the heme-related or hemoprotein-related disorder and having received the treatment.

Such applications correspond to many possible applications among which some will be described in what follows.

Notably, the method may be used in :

- the monitoring of the treatment of heme arginate porphyries ;
- the monitoring of a purified or recombinant hemopexin treatment ;
- monitoring of an addition of a blood substitute derived from Hb or other therapeutic use;
- monitoring of treatments that induce hemolysis such as ecmo (extra-corporal circulation, hemodialysis or cardiac prostheses) ;
- characterizing plasma exchanges, plasmapheresis (clearance of Hb and heme by addition of their scavengers haptoglobin and hemopexin) calculation of the exchange yield (dilution of the patient's plasma), and
- using the determination of hemopexin to interact with pathologies of the red globule.

The Applicant has also carried out experiments on patients with Sickle cell disease (SCD) and beta-thalassemias by using the previously described method. The major part of the patients is under treatment HU, transfusion or both (other treatments such as chelators iron are associated).

In broad outline knowing when the absence of treatments all parameters of hemolysis / dyserythropoiesis would then be higher, the Applicant has been able to detect a higher Hb content plasma in SCD only and closer to the normal range for beta- thalassemias (intermediate or under transfusion program). Especially this Hb is mostly free and not complexed with haptoglobin in SCD. It will therefore be able to extravasate in tissues and degrade with heme accumulation at the membrane (glomeruli, for example) and ultimately induce dysfunction organs in the long term but also catabolize part of the NO synthesized by endothelial cells (promoting vasoconstriction). The metHb content can be linked to an oxidative stress and to the level of vasoconstriction (hypertension) after NO reaction with oxyHb.

In the context of beta-thalassemia, it is the heme that is the majority. The degradation of heme in the plasma will induce also prooxidant cytotoxic reactions after release of the iron if not supported by heme oxygenase (ferritin). If a part of the heme is catabolized in the endothelium, there will be an activation of signaling pathways may be favorable across the degradation products of heme: biliverdin and bilirubin but especially CO (anti-inflammatory, anti-aggregation, anti-proliferative, vasoactive ...). The accumulation at the membrane will induce necrosis (treatment of heme arginate). The Applicant has also noticed the separation of measurement intervals from biomarkers according to pathologies.

The important quantity of heme in beta-thalassemia, and notably for transfused patients, with a depletion of hemopexin was totally unexpected and has been detected thanks to the precision of the previously described determining method.

Another application of the determining method is for the monitoring of delayed hemolytic transfusion reaction (DHTR) which is a type of transfusion reaction.

There again, the inventors have shown that using the determining method enables to form a better diagnostic of a DHTR.

Finally the Applicant has shown the steady-state of an untreated patient through the status of these biomarkers. There is no measurement bias or problem in managing the samples making the data little reliable. So the follow-up of a treatment can use these biomarkers in the hope of a favorable evolution.

In addition, as applications, one may consider studying specifically the Hx-heme complex. For example, one may consider increasing HO activity in tissues that have receptors of the Hx-heme complex in order to overcome the presence of oxidative stress. As another example, Hx-heme complex may be used as an adjuvant to an organ conditioning solution (kidney, liver, etc.) before a transplant to lessen the deleterious effects of an ischemia/reperfusion syndrome. The use of heme as a biological messenger is possible especially since its follow-up would be made possible by using the dosage of this complex.

It may also be consider HO for heme oxygenase with activation of the antioxidation pathways.

To conclude, it has been proposed a method for determining at least one protein in the blood or its metabolized/degradation products (natural pigments), which is more precise while still being simple to implement. This enables to consider many applications for heme-related or hemoprotein-related disorders or diseases.

This precision can notably be increased by considering one of the following chemical dosing techniques:

- dosing oxyhemoglobin by adding CO,
- dosing methemoglobin by adding KCN or $N_3^-$,
- dosing plasma heme by adding CO and sodium dithionite,
- dosing heme bound to hemopexin by adding DTN,
- dosing total hemopexin by adding heme, and
- dosing total hemopexin by adding heme and sodium dithionite.

**Claims**

1. A method for determining at least one content in protein in a biological sample, the biological sample comprising several proteins among which oxyhemoglobin, methemoglobin, heme bound to serum albumin, heme bound to hemopexin and bilirubin, the method for determining at least comprising the steps of:

   a) providing an initial spectrum of the sample on a wavelength range of interest,
   b) setting the current spectrum as the initial spectrum,
   c) iterating the following steps:

   c1) determining the content in a protein in the sample, the determination being carried out by using a spectral analysis of the current spectrum and optionally a chemical dosing,
   c2) deducing the overall spectral component on the wavelength range of interest associated to the protein based on the determined content for said protein, and
   c3) removing the deduced spectral component from the current spectrum,

   d) outputting the determined contents in protein,

   step c) being iterated successively for different proteins as long as the content in oxyhemoglobin, methemoglobin, heme bound to serum albumin and hemopexin and bilirubin are not determined; wherein the spectral analysis comprises:

   - providing a reference spectrum associated with the considered protein, and
   - calculating a normalization coefficient between the reference spectrum and the current spectrum, said normalization coefficient being the ratio between the value of second derivative of the considered protein and the value of second derivative of the reference spectrum, with the value of second derivative of a protein being defined as the maximum amplitude over the wavelength range of interest of the second derivative with wavelength of the spectrum of the spectral component of the protein in the sample or the maximum value over the wavelength range of interest of an absolute value of the difference between a minima and a maxima of the second derivative with wavelength of the spectrum of the spectral component of the protein in the sample, and

   the value of second derivative of the reference spectrum being defined as the maximum amplitude over the wavelength range of interest of the second derivative with wavelength of the reference spectrum or the maximum value over the wavelength range of interest of an absolute value of the difference between a minima and a maxima of the second derivative with wavelength of the reference spectrum; and
   wherein, when chemical dosing is applied, it is chosen in the group consisting in:

   - dosing oxyhemoglobin by adding CO,
   - dosing methemoglobin by adding KCN,
   - dosing plasma heme by adding CO and sodium dithionite,
   - dosing heme bound to hemopexin by adding sodium dithionite,
   - dosing total hemopexin by adding heme,
   - dosing total hemopexin by adding heme and sodium dithionite, and
   - dosing total hemopexin by adding heme, CO and sodium dithionite.

2. The method for determining according to claim **1,** wherein step c) is iterated by decreasing values of second derivatives of the proteins.

3. The method for determining according to claim **1** or **2**, wherein at the least one of the following properties is fulfilled:

- a spectral analysis is carried out for the oxyhemoglobin and the normalization coefficient is calculated for at least one wavelength chosen in three intervals, the first interval encompassing wavelengths comprised between 415 nanometers and 417 nanometers, the second interval encompassing wavelengths comprised between 542 nanometers and 544 nanometers and a third interval encompassing wavelengths comprised between 576 nanometers and 578 nanometers, the interval being preferably the third interval;

- a spectral analysis is carried out for the methemoglobin and the normalization coefficient is calculated for at least one wavelength chosen in the range of 350 nanometers to 750 nanometers, and

- a spectral analysis is carried out for the bilirubin and the normalization coefficient is calculated for at least one wavelength chosen in the range of 350 nanometers to 750 nanometers.

4. The method for determining according to any one of claims **1** to **3**, wherein a spectral analysis is carried out for the plasma heme and the normalization coefficient is calculated for at least one wavelength chosen in the range of 300 nanometers to 750 nanometers using heme bound to serum albumin and heme bound to hemopexin reference spectra.

5. The method for determining according to any one of claims **1** to **4**, wherein at the step for outputting, the determined contents comprise additional contents in protein which are different from the content in oxyhemoglobin, from the content in methemoglobin, from the content in heme bound to serum albumin and hemopexin from the content in bilirubin, the additional contents being the contents of proteins chosen in the group consisting of:

- carboxylated hemoglobin,
- myoglobin,
- porphyrins,
- porphobilinogen,
- urobillin,
- products of catabolism of heme by heme oxygenase, and
- products of bilirubin degradation and oxidation, such as biliverdin or heme boxes,
- products of organ dysfunction and cytosis,
- metabolic substrates, and
- metabolic degradation products.

6. The method for determining according to any one of claims **1** to **5**, wherein when the content in oxyhemoglobin, methemoglobin, heme bound to serum albumin and bilirubin are successively determined, at least one of oxyhemoglobin, methemoglobin, heme bound to serum albumin content and bilirubin is determined by chemical dosing.

7. Use of the method for determining according to any one of claims **1** to **6** in:

• a method for predicting that a subject is at risk of suffering from a heme-related or hemoprotein-related disorder, the method for predicting at least comprising the step of:

- carrying out the steps of a method for determining at least one content in protein in a biological sample of the subject, to obtain determined parameters, the method for determining being according to any one of claims 1 to 6,
- predicting that the subject is at risk of suffering from the heme-related or hemoprotein-related disorder on the determined parameters;

• a method for diagnosing a heme-related or hemoprotein-related disorder, the method for diagnosing at least comprising the step of:

- carrying out the steps of a method for determining at least one content in protein in a biological sample of the subject, to obtain determined contents in protein, the method for determining being according to any one of claims 1 to 6, and
- diagnosing the heme-related or hemoprotein-related disorder based on the determined contents in protein;

• a method for defining stages of a heme-related or hemoprotein-related disorder, the method for defining at least comprising the step of:

- carrying out the steps of a method for determining at least one content in protein in a biological sample of the subject, to obtain determined contents in protein, the method for determining being according to any one of claims 1 to 6, and
- defining the stages of the heme-related or hemoprotein-related disorder based on the determined contents in protein;

• a method for identifying a therapeutic target for preventing and/or treating a heme-related or hemoprotein-related disorder, the method comprising the steps of:

- carrying out the steps of a method for determining at least one content in protein in a biological sample of images of a first subject, to obtain first determined contents in protein, the method for determining being according to any one of claims 1 to 6 and the first subject being a subject suffering from the heme-related or hemoprotein-related disorder,
- carrying out the steps of the method for determining at least one content in protein in a biological sample of a second subject, to obtain second determined contents in protein, the method for determining being according to any one of claims 1 to 6 and the second subject being a subject not suffering from the heme-related or hemoprotein-related disorder, and
- selecting a therapeutic target based on the comparison of the first and second determined contents in protein;

• a method for identifying a biomarker, the biomarker being a diagnostic biomarker of a heme-related or hemoprotein-related disorder, a susceptibility biomarker of a heme-related or hemoprotein-related disorder, a prognostic biomarker of a heme-related or hemoprotein-related disorder or a predictive biomarker in response to the treatment of a heme-related or hemoprotein-related disorder, the method comprising the steps of:

- carrying out the steps of a method for determining at least one content in protein in a biological sample of a first subject, to obtain first determined contents, the method for determining being according to any one of claims 1 to 6 and the first subject being a subject suffering from the heme-related or hemoprotein-related disorder,
- carrying out the steps of the method for determining at least one content in protein in a biological sample of images of a second subject, to obtain second determined contents in protein, the method for determining being according to any one of claims 1 to 6 and the second subject being a subject not suffering from the heme-related or hemoprotein-related disorder, and
- selecting a biomarker based on the comparison of the first and second determined contents in protein, and

• a method for screening a compound useful as a medicine, the compound having an effect on a known therapeutical target, for preventing and/or treating a heme-related or hemoprotein-related disorder, the method comprising the steps of:

- carrying out the steps of a method for determining at least one content in protein in a biological sample of a first subject, to obtain first determined contents in protein, the method for determining being according to any one of claims 1 to 6 and the first subject being a subject suffering from the heme-related or hemoprotein-related disorder and having received the compound,
- carrying out the steps of the method for determining at least one content in protein in a biological sample of a second subject, to obtain second determined contents in protein, the method for determining being according to any one of claims 1 to 6 and the second subject being a subject suffering from the heme-related or hemoprotein-related disorder and not having received the compound, and
- selecting a compound based on the comparison of the first and second determined contents in protein.

8. A method for qualifying or disqualifying medical bags containing a biological sample of subject, notably blood bags, the method comprising:

- carrying out the steps of a method for determining at least one content in protein in the medical bag, to obtain determined contents in protein, the method for determining being according to any one of claims 1 to 6,
- qualifying or disqualifying medical bags based on the determined contents in protein.

9. A method for monitoring a treatment against heme-related or hemoprotein-related disorder in a subject suffering from the heme-related or hemoprotein-related disorder and having received the treatment, the method comprising:

- carrying out the steps of a method for determining at least one content in protein in a biological sample of the subject, to obtain determined contents in protein, the method for determining being according to any one of claims 1 to 6, and
- monitoring the determined contents in protein with monitoring a treatment against heme-related or hemoprotein-related disorder in a subject suffering from the heme-related or hemoprotein-related disorder and having received the treatment.

10. A computer program product comprising a computer readable medium, having thereon a computer program comprising program instructions, the computer program being loadable into a data-processing unit and adapted to cause execution of the steps of a method according to any one of claims 1 to 9 when the computer program is run by the data processing unit.

11. A computer readable medium having encoded thereon a computer program according to claim **10.**

12. An apparatus (10) for determining at least one content in protein in a biological sample (20), several proteins among which oxyhemoglobin, methemoglobin, heme bound to serum albumin, heme bound to hemopexin and bilirubin, the apparatus (10) for determining at least comprising a spectrophotometer (14), dosing materials (16) and an analysis system (18), the apparatus (10) being adapted to carry out one method according to any one of claims **1** to 9.

**Patentansprüche**

1. Verfahren zum Bestimmen mindestens eines Proteingehalts in einer biologischen Probe, wobei die biologische Probe mehrere Proteine umfasst, darunter Oxyhämoglobin, Methämoglobin, an Serumalbumin gebundenes Häm, an Hämopexin gebundenes Häm und Bilirubin, wobei das Verfahren zum Bestimmen mindestens die folgenden Schritte umfasst:

a) Bereitstellen eines Anfangsspektrums der Probe in einem interessierenden Wellenlängenbereich,
b) Einstellen des aktuellen Spektrums als Anfangsspektrum,
c) Iterieren der folgenden Schritte:

c1) Bestimmen des Gehalts eines Proteins in der Probe, wobei die Bestimmung unter Verwendung einer Spektralanalyse des aktuellen Spektrums und gegebenenfalls einer chemischen Dosierung ausgeführt wird,
c2) Ableiten der gesamten Spektralkomponente im interessierenden Wellenlängenbereich, die dem Protein zugeordnet ist, auf Basis des bestimmten Gehalts für das Protein, und
c3) Entfernen der abgeleiteten Spektralkomponente aus dem aktuellen Spektrum,

d) Ausgeben der bestimmten Proteingehalte,

wobei Schritt c) nacheinander für unterschiedliche Proteine iteriert wird, solange der Gehalt an Oxyhämoglobin, Methämoglobin, an Serumalbumin und Hämopexin gebundenem Häm und Bilirubin nicht bestimmt wurde; wobei
die Spektralanalyse umfasst:

- Bereitstellen eines Referenzspektrums, das dem betrachteten Protein zugeordnet ist, und
- Berechnen eines Normalisierungskoeffizienten zwischen dem Referenzspektrum und dem aktuellen Spektrum, wobei der Normalisierungskoeffizient das Verhältnis zwischen dem Wert der zweiten Ableitung des betrachteten Proteins und dem Wert der zweiten Ableitung des Referenzspektrums ist, wobei der Wert der zweiten Ableitung eines Proteins als maximale Amplitude über den interessierenden Wellenlängenbereich der zweiten Ableitung mit Wellenlänge des Spektrums der Spektralkomponente des Proteins in der Probe oder maximaler Wert über den interessierenden Wellenlängenbereich eines Absolutwerts der Differenz zwischen einem Minimum und einem Maximum der zweiten Ableitung mit Wellenlänge des Spektrums der Spektralkomponente des Proteins in der Probe definiert ist, und

wobei der Wert der zweiten Ableitung des Referenzspektrums als maximale Amplitude über den interessierenden Wellenlängenbereich der zweiten Ableitung mit Wellenlänge des Referenzspektrums oder maximaler Wert über den interessierenden Wellenlängenbereich eines Absolutwerts der Differenz zwischen einem Minimum und einem Maximum der zweiten Ableitung mit Wellenlänge des Referenzspektrums definiert ist; und

wobei, wenn chemische Dosierung angewendet wird, diese ausgewählt ist aus der Gruppe bestehend aus:

- Dosierung von Oxyhämoglobin durch Zugabe von CO,
- Dosierung von Methämoglobin durch Zugabe von KCN,
- Dosierung von Plasma-Häm durch Zugabe von CO und Natriumdithionit,
- Dosierung von an Hämopexin gebundenem Häm durch Zugabe von Natriumdithionit,
- Dosierung von Gesamthämopexin durch Zugabe von Häm,
- Dosierung von Gesamthämopexin durch Zugabe von Häm und Natriumdithionit, und
- Dosierung von Gesamthämopexin durch Zugabe von Häm, CO und Natriumdithionit.

2. Verfahren zum Bestimmen nach Anspruch **1**, wobei Schritt c) nach abnehmenden Werten von zweiten Ableitungen der Proteine iteriert wird.

3. Verfahren zum Bestimmen nach Anspruch **1** oder **2**, wobei mindestens eine der folgenden Eigenschaften erfüllt ist:

- es wird eine Spektralanalyse für das Oxyhämoglobin ausgeführt und der Normalisierungskoeffizient für mindestens eine Wellenlänge, ausgewählt in drei Intervallen, berechnet, wobei das erste Intervall Wellenlängen im Bereich zwischen 415 Nanometer und 417 Nanometer beinhaltet, das zweite Intervall Wellenlängen im Bereich zwischen 542 Nanometer und 544 Nanometer beinhaltet, und ein drittes Intervall Wellenlängen im Bereich zwischen 576 Nanometer und 578 Nanometer beinhaltet, wobei das Intervall vorzugsweise das dritte Intervall ist;
- es wird eine Spektralanalyse für das Methämoglobin ausgeführt und der Normalisierungskoeffizient für mindestens eine Wellenlänge, ausgewählt im Bereich von 350 Nanometer bis 750 Nanometer, berechnet und
- es wird eine Spektralanalyse für das Bilirubin ausgeführt und der Normalisierungskoeffizient für mindestens eine Wellenlänge, ausgewählt im Bereich von 350 Nanometer bis 750 Nanometer, berechnet.

4. Verfahren zum Bestimmen nach einem der Ansprüche **1** bis **3**, wobei eine Spektralanalyse für das Plasma-Häm ausgeführt und der Normalisierungskoeffizient für mindestens eine Wellenlänge, ausgewählt im Bereich von 300 Nanometer bis 750 Nanometer, unter Verwendung von an Serumalbumin gebundenem Häm und an Hämopexin gebundenem Häm als Referenzspektren berechnet wird.

5. Verfahren zum Bestimmen nach einem der Ansprüche 1 bis 4, wobei beim Schritt zum Ausgeben die bestimmten Gehalte zusätzliche Proteingehalte umfassen, die sich vom Gehalt an Oxyhämoglobin, vom Gehalt an Methämoglobin, vom Gehalt an an Serumalbumin und Hämopexin gebundenem Häm, vom Gehalt an Bilirubin unterscheiden, wobei die zusätzlichen Gehalte die Gehalte von Proteinen sind, ausgewählt aus der Gruppe bestehend aus:

- carboxyliertem Hämoglobin,
- Myoglobin,
- Porphyrinen,
- Porphobilinogen,
- Urobillin,
- Produkten des Katabolismus von Häm durch Hämoxygenase, und
- Produkten des Abbaus und der Oxidation von Bilirubin, wie etwa Biliverdin oder Häm-Boxen,
- Produkten von Organdysfunktion und Zytose,
- Stoffwechselsubstraten und
- Stoffwechselabbauprodukten.

6. Verfahren zum Bestimmen nach einem der Ansprüche **1** bis **5,** wobei, wenn der Gehalt an Oxyhämoglobin, Methämoglobin, an Serumalbumin gebundenem Häm und Bilirubin nacheinander bestimmt wird, mindestens eines von Oxyhämoglobin, Methämoglobin, Gehalt von an Serumalbumin gebundenem Häm und Bilirubin durch chemische Dosierung bestimmt wird.

7. Verwendung des Verfahrens zum Bestimmen nach einem der Ansprüche 1 bis 6 in:

- einem Verfahren zum Vorhersagen, dass ein Subjekt gefährdet ist, an einer häm-bezogenen oder hämoprotein-bezogenen Störung zu leiden, wobei das Verfahren zum Vorhersagen mindestens den folgenden Schritt umfasst:

- Ausführen der Schritte eines Verfahrens zum Bestimmen mindestens eines Proteingehalts in einer biologischen Probe des Subjekts, um bestimmte Parameter zu erhalten, wobei das Verfahren zum Bestimmen nach einem der Ansprüche **1** bis **6** ist,
- Vorhersagen, dass das Subjekt gefährdet ist, an der häm-bezogenen oder hämoprotein-bezogenen Störung zu leiden, anhand der bestimmten Parameter;

- einem Verfahren zum Diagnostizieren einer häm-bezogenen oder hämoprotein-bezogenen Störung, wobei das Verfahren zum Diagnostizieren mindestens den folgenden Schritt umfasst:

- Ausführen der Schritte eines Verfahrens zum Bestimmen mindestens eines Proteingehalts in einer biologischen Probe des Subjekts, um bestimmte Proteingehalte zu erhalten, wobei das Verfahren zum Bestimmen nach einem der Ansprüche **1** bis **6** ist, und
- Diagnostizieren der häm-bezogenen oder hämoprotein-bezogenen Störung auf Basis der bestimmten Proteingehalte;

- einem Verfahren zum Definieren von Stadien einer häm-bezogenen oder hämoprotein-bezogenen Störung, wobei das Verfahren zum Definieren mindestens den folgenden Schritt umfasst:

- Ausführen der Schritte eines Verfahrens zum Bestimmen mindestens eines Proteingehalts in einer biologischen Probe des Subjekts, um bestimmte Proteingehalte zu erhalten, wobei das Verfahren zum Bestimmen nach einem der Ansprüche **1** bis **6** ist, und
- Definieren der Stadien der häm-bezogenen oder hämoprotein-bezogenen Störung auf Basis der bestimmten Proteingehalte;

- einem Verfahren zum Identifizieren eines therapeutischen Ziels zum Vorbeugen und/oder Behandeln einer häm-bezogenen oder hämoprotein-bezogenen Störung, wobei das Verfahren die folgenden Schritte umfasst:

- Ausführen der Schritte eines Verfahrens zum Bestimmen mindestens eines Proteingehalts in einer biologischen Probe von Bildern eines ersten Subjekts, um erste bestimmte Proteingehalte zu erhalten, wobei das Verfahren zum Bestimmen nach einem der Ansprüche **1** bis **6** ist und das erste Subjekt ein Subjekt ist, das an der häm-bezogenen oder hämoprotein-bezogenen Störung leidet,
- Ausführen der Schritte des Verfahrens zum Bestimmen mindestens eines Proteingehalts in einer biologischen Probe eines zweiten Subjekts, um zweite bestimmte Proteingehalte zu erhalten, wobei das Verfahren zum Bestimmen nach einem der Ansprüche **1** bis **6** ist und das zweite Subjekt ein Subjekt ist, das nicht an der häm-bezogenen oder hämoprotein-bezogenen Störung leidet, und
- Auswählen eines therapeutischen Ziels auf Basis des Vergleichs der ersten und der zweiten bestimmten Proteingehalte;

- einem Verfahren zum Identifizieren eines Biomarkers, wobei der Biomarker ein diagnostischer Biomarker einer häm-bezogenen oder hämoprotein-bezogenen Störung, ein Anfälligkeits-Biomarker einer häm-bezogenen oder hämoprotein-bezogenen Störung, ein prognostischer Biomarker einer häm-bezogenen oder hämoprotein-bezogenen Störung oder ein prädiktiver Biomarker in Reaktion auf die Behandlung einer häm-bezogenen oder hämoprotein-bezogenen Störung ist, wobei das Verfahren die folgenden Schritte umfasst:

- Ausführen der Schritte eines Verfahrens zum Bestimmen mindestens eines Proteingehalts in einer biologischen Probe eines ersten Subjekts, um erste bestimmte Gehalte zu erhalten, wobei das Verfahren zum Bestimmen nach einem der Ansprüche **1** bis **6** ist und das erste Subjekt ein Subjekt ist, das an der häm-bezogenen oder hämoprotein-bezogenen Störung leidet,
- Ausführen der Schritte des Verfahrens zum Bestimmen mindestens eines Proteingehalts in einer biologischen Probe von Bildern eines zweiten Subjekts, um zweite bestimmte Proteingehalte zu erhalten, wobei das Verfahren zum Bestimmen nach einem der Ansprüche **1** bis **6** ist und das zweite Subjekt ein Subjekt ist, das nicht an der häm-bezogenen oder hämoprotein-bezogenen Störung leidet, und
- Auswählen eines Biomarkers auf Basis des Vergleichs der ersten und der zweiten bestimmten Proteingehalte, und

- einem Verfahren zum Screening einer Verbindung, die als Arzneimittel nützlich ist, wobei die Verbindung eine Wirkung auf ein bekanntes therapeutisches Ziel aufweist, zum Vorbeugen und/oder Behandeln einer häm-bezogenen oder hämoprotein-bezogenen Störung, wobei das Verfahren die folgenden Schritte umfasst:

- Ausführen der Schritte eines Verfahrens zum Bestimmen mindestens eines Proteingehalts in einer biologischen Probe eines ersten Subjekts, um erste bestimmte Proteingehalte zu erhalten, wobei das Verfahren zum Bestimmen nach einem der Ansprüche **1** bis **6** ist und das erste Subjekt ein Subjekt ist, das an der häm-bezogenen oder hämoprotein-bezogenen Störung leidet und die Verbindung erhalten hat,
- Ausführen der Schritte des Verfahrens zum Bestimmen mindestens eines Proteingehalts in einer biologischen Probe eines zweiten Subjekts, um zweite bestimmte Proteingehalte zu erhalten, wobei das Verfahren zum Bestimmen nach einem der Ansprüche **1** bis **6** ist und das zweite Subjekt ein Subjekt ist, das an der häm-bezogenen oder hämoprotein-bezogenen Störung leidet und die Verbindung nicht erhalten hat, und
- Auswählen einer Verbindung auf Basis des Vergleichs der ersten und der zweiten bestimmten Proteingehalte.

**8.** Verfahren zum Qualifizieren oder Disqualifizieren von medizinischen Beuteln, die eine biologische Probe eines Subjekts enthalten, insbesondere Blutbeuteln, wobei das Verfahren umfasst:

- Ausführen der Schritte eines Verfahrens zum Bestimmen mindestens eines Proteingehalts in dem medizinischen Beutel, um bestimmte Proteingehalte zu erhalten, wobei das Verfahren zum Bestimmen nach einem der Ansprüche **1** bis **6** ist,
- Qualifizieren oder Disqualifizieren von medizinischen Beuteln auf Basis der bestimmten Proteingehalte.

**9.** Verfahren zum Überwachen einer Behandlung einer häm-bezogenen oder hämoprotein-bezogenen Störung bei einem Subjekt, das an der häm-bezogenen oder hämoprotein-bezogenen Störung leidet und die Behandlung erhalten hat, wobei das Verfahren umfasst:

- Ausführen der Schritte eines Verfahrens zum Bestimmen mindestens eines Proteingehalts in einer biologischen Probe des Subjekts, um bestimmte Proteingehalte zu erhalten, wobei das Verfahren zum Bestimmen nach einem der Ansprüche **1** bis **6** ist, und
- Überwachen der bestimmten Proteingehalte mit Überwachen einer Behandlung einer häm-bezogenen oder hämoprotein-bezogenen Störung bei einem Subjekt, das an der häm-bezogenen oder hämoprotein-bezogenen Störung leidet und die Behandlung erhalten hat.

**10.** Computerprogrammprodukt, das ein computerlesbares Medium umfasst, auf dem sich ein Computerprogramm befindet, das Programmanweisungen umfasst, wobei das Computerprogramm in eine Datenverarbeitungseinheit geladen werden kann und geeignet ist, Ausführung der Schritte eines Verfahrens nach einem der Ansprüche **1** bis **9** zu veranlassen, wenn das Computerprogramm von der Datenverarbeitungseinheit ausgeführt wird.

**11.** Computerlesbares Medium, auf dem ein Computerprogramm nach Anspruch **10** codiert ist.

**12.** Einrichtung (10) zum Bestimmen mindestens eines Proteingehalts in einer biologischen Probe (20), mehrerer Proteine, darunter Oxyhämoglobin, Methämoglobin, an Serumalbumin gebundenes Häm, an Hämopexin gebundenes Häm und Bilirubin, wobei die Einrichtung (10) zum Bestimmen mindestens ein Spektralphotometer (14), Dosiermaterialien (16) und ein Analysesystem (18) umfasst, wobei die Einrichtung (10) geeignet ist, ein Verfahren nach einem der Ansprüche **1** bis **9** auszuführen.

## Revendications

**1.** Un procédé de détermination d'au moins un contenu protéique dans un échantillon biologique, l'échantillon biologique comprenant plusieurs protéines dont l'oxyhémoglobine, la méthémoglobine, l'hème lié à l'albumine sérique, l'hème lié à l'hémopexine et la bilirubine, le procédé de détermination comprenant au moins les étapes suivantes :

a) fournir un spectre initial de l'échantillon sur une plage de longueurs d'onde d'intérêt,
b) définir le spectre actuel comme spectre initial,
c) itérer les étapes suivantes :

c1) déterminer la teneur en protéines de l'échantillon, la détermination se faisant via une analyse spectrale du spectre actuel et, éventuellement, un dosage chimique.
c2) déduire la composante spectrale globale sur la gamme de longueur d'onde d'intérêt associée à la protéine sur la base de la teneur déterminée pour ladite protéine, et

c3) supprimer la composante spectrale déduite du spectre actuel,

d) restitué les contenus déterminés en protéines.

l'étape c) étant itérée successivement pour différentes protéines tant que le contenu en oxyhémoglobine, méthémoglobine, hème lié à l'albumine sérique et à l'hémopexine et la bilirubine n'est pas déterminée ;

dans lequel l'analyse spectrale comprend

- fournir un spectre de référence associé à la protéine considérée, et

- calculer un coefficient de normalisation entre le spectre de référence et le spectre actuel, ledit coefficient de normalisation étant le rapport entre la valeur de la dérivée seconde de la protéine considérée et la valeur de la dérivée seconde du spectre de référence, avec la valeur de la dérivée seconde d'une protéine étant définie comme l'amplitude maximale sur la gamme de longueurs d'onde d'intérêt de la dérivée seconde avec la longueur d'onde du spectre de la composante spectrale de la protéine dans l'échantillon ou la valeur maximale sur la gamme de longueurs d'onde d'intérêt d'une valeur absolue de la différence entre un minima et un maxima de la dérivée seconde avec la longueur d'onde du spectre de la composante spectrale de la protéine dans l'échantillon, et la valeur de la dérivée seconde du spectre de référence étant définie comme l'amplitude maximale sur la gamme de longueurs d'onde d'intérêt de la dérivée seconde avec la longueur d'onde du spectre de référence ou la valeur maximale sur la gamme de longueurs d'onde d'intérêt d'une valeur absolue de la différence entre un minima et un maxima de la dérivée seconde avec la longueur d'onde du spectre de référence ; et

dans lequel, lorsque le dosage chimique est appliqué, il est choisi dans le groupe constitué par :

- dosage de l'oxyhémoglobine par addition de CO,
- dosage de la méthémoglobine par addition de KCN,
- dosage de l'hème plasmatique par addition de CO et de dithionite de sodium,
- dosage de l'hème lié à l'hémopexine par addition de dithionite de sodium,
- dosage de l'hémopexine totale par addition d'hème,
- dosage de l'hémopexine totale par addition d'hème et de dithionite de sodium, et
- dosage de l'hémopexine totale par addition d'hème, de CO et de dithionite de sodium.

2. Le procédé de détermination selon la revendication **1,** dans lequel l'étape c) est itérée par valeurs décroissantes des dérivées secondes des protéines.

3. Le procédé de de détermination selon la revendication **1** ou **2,** dans lequel au moins l'une des propriétés suivantes est remplie :

- une analyse spectrale est effectuée pour l'oxyhémoglobine et le coefficient de normalisation est calculé pour au moins une longueur d'onde choisie dans trois intervalles, le premier intervalle englobe les longueurs d'onde comprises entre 415 nanomètres et 417 nanomètres, le deuxième intervalle englobant les longueurs d'onde comprises entre 542 nanomètres et 544 nanomètres et un troisième intervalle englobant les longueurs d'onde comprise entre 576 nanomètres et 578 nanomètres, l'intervalle étant de préférence le troisième intervalle ;
- une analyse spectrale est effectuée pour la méthémoglobine et le coefficient de normalisation est calculé pour au moins une longueur d'onde choisie dans l'intervalle de 350 nanomètres à 750 nanomètres, et
- une analyse spectrale est effectuée pour la bilirubine et le coefficient de normalisation est calculé pour au moins une longueur d'onde choisie dans l'intervalle de 350 nanomètres à 750 nanomètres.

4. Le procédé de détermination selon l'une quelconque des revendications **1** à **3,** dans lequel une analyse spectrale est effectuée pour l'hème plasmatique et le coefficient de normalisation est calculé pour au moins une longueur d'onde choisie dans l'intervalle de 300 nanomètres à 750 nanomètres en utilisant les spectres de référence de l'hème lié à l'albumine sérique et de l'hème lié à l'hémopexine.

5. Le procédé de détermination selon l'une quelconque des revendications **1** à **4,** dans lequel, à l'étape de restitution, les contenus déterminés comprennent des contenus additionnels en protéines qui sont différents du contenu en oxyhémoglobine, du contenu en méthémoglobine, du contenu en hème lié à l'albumine sérique et à l'hémopexine, du contenu en bilirubine, les contenus supplémentaires étant les contenu protéiques choisis dans le groupe consistant en :

- l'hémoglobine carboxylée,
- la myoglobine,
- la porphyrines,
- le porphobilinogène,
- l'urobilline,
- les produits du catabolisme de l'hème par l'hème oxygénase, et
- les produits de la dégradation et de l'oxydation de la bilirubine, tels que la biliverdine ou les boîtes d'hème,
- les produits du dysfonctionnement des organes et de la cytose,
- les substrats métaboliques et
- les produits de dégradation métabolique.

6. Le procédé de détermination selon l'une quelconque des revendications **1** à **5,** dans lequel, lors de la détermination successive des contenus en oxyhémoglobine, méthémoglobine, hème lié à l'albumine sérique et bilirubine, au moins un des contenus en oxyhémoglobine, méthémoglobine, hème lié à l'albumine sérique et bilirubine est déterminé par dosage chimique.

7. Utilisation du procédé de détermination selon l'une quelconque des revendications **1** à **6** dans :

• un procédé pour prédire qu'un sujet risque de souffrir d'un trouble lié à l'hème ou à l'hémoprotéine, le procédé de prédiction comprenant au moins l'étape consistant à :

- mettre en oeuvre les étapes d'un procédé de détermination d'au moins un contenu protéique dans un échantillon biologique du sujet, pour obtenir des paramètres déterminés, le procédé de détermination étant selon l'une quelconque des revendications **1** à **6,**
- prédire que le sujet risque de souffrir du troubles lié à l'hème ou à l'hémoprotéine sur la base des paramètres déterminés ;

• un procédé de diagnostic d'un trouble lié à l'hème ou à l'hémoprotéine, le procédé de diagnostic comprenant au moins l'étape consistant à :

- mettre en oeuvre les étapes d'un procédé de détermination d'au moins un contenu en protéique dans un échantillon biologique du sujet, pour obtenir des contenues protéiques déterminés, le procédé de diagnostic étant selon l'une quelconque des revendications **1** à **6,** et
- diagnostiquer le trouble lié à l'hème ou à l'hémoprotéine sur la base des contenus protéiques déterminés ;

• un procédé pour définir les stades d'un trouble lié à l'hème ou à l'hémoprotéine, le procédé de définition comprenant au moins l'étape consistant à :

- mettre en oeuvre les étapes du procédé de détermination d'au moins un contenu protéique dans un échantillon biologique du sujet, pour obtenir des contenus protéiques déterminés, le procédé de détermination étant selon l'une quelconque des revendications **1** à **6,** et
- définir les stades du trouble lié à l'hème ou à l'hémoprotéine sur la base du contenu en protéines déterminées ;

• un procédé d'identification d'une cible thérapeutique pour la prévention et/ou le traitement d'un trouble lié à l'hème ou à l'hémoprotéine, le procédé comprenant les étapes suivantes :

- mettre en oeuvre les étapes du procédé de détermination d'au moins un contenu protéique dans un échantillon biologique d'images d'un premier sujet, pour obtenir des premiers contenus en protéines déterminés, le procédé de détermination étant selon l'une quelconque des revendications **1** à **6** et le premier sujet étant un sujet souffrant de trouble lié à l'hème ou à l'hémoprotéine,
- mettre en oeuvre les étapes du procédé de détermination d'au moins un contenu protéique dans un échantillon biologique d'un second sujet, pour obtenir les seconds contenus protéiques déterminés, le procédé de détermination étant selon l'une quelconque des revendications **1** à **6** et le premier sujet étant un sujet souffrant de trouble lié à l'hème ou à l'hémoprotéine selon l'une quelconque des revendications **1** à **6** et le second sujet étant un sujet ne souffrant pas de troubles liés à l'hème ou à l'hémoprotéine, et
- sélectionner une cible thérapeutique sur la base de la comparaison des premiers et seconds contenus protéiques déterminés;

• un procédé d'identification d'un biomarqueur, le biomarqueur étant un biomarqueur de diagnostic d'une trouble lié à l'hème ou à l'hémoprotéine, un biomarqueur de susceptibilité d'une trouble lié à l'hème ou à l'hémoprotéine, un biomarqueur pronostique d'une trouble lié à l'hème ou à l'hémoprotéine ou un biomarqueur prédictif en réponse au traitement d'une trouble lié à l'hème ou à l'hémoprotéine le procédé comprenant les étapes consistant à :

- mettre en oeuvre les étapes d'un procédé de détermination d'au moins un contenu protéique dans un échantillon biologique d'un premier sujet, afin d'obtenir des premier contenus déterminés, le procédé de détermination étant selon l'une quelconque des revendications **1** à **6** et le premier sujet étant un sujet souffrant du trouble lié à l'hème ou à l'hémoprotéine,
- la mise en oeuvre des étapes du procédé de détermination d'au moins un contenu protéique dans un échantillon biologique d'images d'un second sujet, pour obtenir les seconds contenus protéiques déterminées, le procédé de détermination étant selon l'une quelconque des revendications **1** à **6** et le second sujet étant un sujet ne souffrant pas du trouble lié à l'hème ou à l'hémoprotéine, et
- sélectionner un biomarqueur sur la base de la comparaison des premiers et deuxièmes contenus en protéines déterminés, et

• un procédé de criblage d'un composé utile comme médicament, le composé ayant un effet sur une cible thérapeutique connue, pour la prévention et/ou le traitement d'un trouble lié à l'hème ou à l'hémoprotéine, la méthode comprenant les étapes suivantes :

- mettre en oeuvre les étapes d'un procédé de détermination d'au moins un contenu protéiques, le procédé de détermination étant selon l'une quelconque des revendications **1** à **6** et le premier sujet étant un sujet souffrant de trouble lié à l'hème ou à l'hémoprotéine,
- mettre en oeuvre les étapes du procédé de détermination d'au moins un contenu protéique dans un échantillon biologique d'un second sujet, pour obtenir des secondes contenus protéiques déterminées, le procédé de détermination étant selon l'une quelconque des revendications **1** à **6** et le second sujet étant un sujet ne souffrant pas de trouble lié à l'hème ou à l'hémoprotéine, et
- sélectionner une cible thérapeutique sur la base de la comparaison des premier et second contenus protéiques déterminées;

**8.** Un procédé de qualification ou de disqualification de poches médicales contenant un échantillon biologique de sujet, notamment de poches de sang, le procédé comprenant

- la mise en oeuvre des étapes d'un procédé de détermination d'au moins un contenu protéique dans la poche médicale, pour obtenir des contenus en protéines déterminées, le procédé de détermination étant selon l'une quelconque des revendications **1** à **6,**
- qualifier ou disqualifier les sacs médicaux sur la base des contenus protéiques déterminés.

**9.** Un procédé de suivi d'un traitement contre un trouble lié à l'hème ou à l'hémoprotéine chez un sujet atteint de trouble lié à l'hème ou à l'hémoprotéine et ayant reçu le traitement, le procédé comprenant :

- mettre en oeuvre les étapes du procédé de détermination d'au moins un contenu protéique dans un échantillon biologique du sujet, pour obtenir des contenus protéinques déterminés, le procédé de détermination étant selon l'une quelconque des revendications **1** à **6,** et
- suivre les contenus protéiques déterminés avec un suivi d'un traitement contre le trouble lié à l'hème ou à l'hémoprotéine chez un sujet souffrant du trouble lié à l'hème ou à l'hémoprotéine et ayant reçu le traitement.

**10.** Un programme d'ordinateur comprenant un support lisible par ordinateur, comportant un programme d'ordinateur comprenant les instructions du programme, le programme d'ordinateur pouvant être chargé dans une unité de traitement de données et étant adapté pour provoquer l'exécution des étapes du procédé selon l'une des revendications **1** à **9** lorsque le programme d'ordinateur est exécuté par l'unité de traitement de données.

**11.** Un support lisible par ordinateur sur lequel est codé un programme d'ordinateur selon la revendication **10.**

**12.** Un appareil (10) pour déterminer au moins un contenu protéique dans un échantillon biologique (20), plusieurs protéines parmi lesquelles l'oxyhémoglobine, la méthémoglobine, l'hème lié à l'albumine sérique, l'hème lié à l'hémopexine et la bilirubine, l'appareil (10) pour la détermination comprenant au moins un spectrophotomètre (14), des matériaux de dosage (16) et un système d'analyse (18), l'appareil (10) étant adapté à la mise en oeuvre d'un procédé selon l'une quelconque des revendications **1** à **9.**

EP 3 887 800 B1

<u>FIG.1</u>

## FIG.2

FIG.3

Absorption spectra for different heme complexes

# FIG.4

Second derivatives for different heme complexes

FIG.5

FIG.6

**EP 3 887 800 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9638686 A1
- US 2007292963 A1
- EP 1211505 A1
- US 2006034730 A1